(19) Europäisches Patentamt
European Patent Office
Office européen des brevets

(11) EP 2 367 535 B1

(12) **EUROPEAN PATENT SPECIFICATION**

(45) Date of publication and mention of the grant of the patent:
**22.02.2017 Bulletin 2017/08**

(21) Application number: **09793490.5**

(22) Date of filing: **25.11.2009**

(51) Int Cl.:
***A61K 31/436*** *(2006.01)* ***A61K 31/337*** *(2006.01)*
***A61K 31/4745*** *(2006.01)* ***A61K 31/704*** *(2006.01)*
***A61K 9/16*** *(2006.01)* ***A61K 9/00*** *(2006.01)*

(86) International application number:
**PCT/EP2009/065857**

(87) International publication number:
**WO 2010/063630 (10.06.2010 Gazette 2010/23)**

(54) **PANCREATIC TUMOUR TREATMENT**

BEHANDLUNG VON BAUCHSPEICHELDRÜSENTUMOREN

TRAITEMENT DE TUMEUR DU PANCRÉAS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.12.2008 EP 08170537**

(43) Date of publication of application:
**28.09.2011 Bulletin 2011/39**

(73) Proprietor: **Biocompatibles UK Ltd.**
**Surrey GU9 8QL (GB)**

(72) Inventors:
- **LEWIS, Andrew, Lennard**
  **Surrey GU9 8QL (GB)**
- **STRATFORD, Peter, William**
  **Surrey GU9 8QL (GB)**
- **FORSTER, Richard, Edward, John**
  **Singapore 129201 (SG)**

(74) Representative: **BTG plc Intellectual Property Group**
**5 Fleet Place**
**London EC4M 7RD (GB)**

(56) References cited:
**EP-A- 1 985 286**
**WO-A-03/020266**
**WO-A-2004/071495**
**WO-A-2005/087193**
**WO-A-2006/027567**
**WO-A-2007/090897**
**WO-A-2007/147902**

- **STATHOPOULOS, G. P. ET AL.: "Treatment of pancreatic cancer with a combination of irinotecan (CPT-11) and gemcitabine: a multicenter phase II study by the Greek Cooperative Group for Pancreatic Cancer" ANNALS OF ONCOLOGY, vol. 14, no. 3, 2003, pages 388-394, XP002585243**
- **OSTER MW ET AL: "Chemotherapy for advanced pancreatic cancer. A comparison of 5-fluorouracil, adriamycin, and mitomycin (FAM) with 5-fluorouracil, streptozotocin, and mitomycin (FSM)." CANCER, vol. 57, no. 1, 1 January 1986 (1986-01-01), pages 29-33, XP002585244**

EP 2 367 535 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of Invention

The present invention relates to compositions for treatment of pancreatic tumours.

Background to Invention

Pancreatic cancer is the fourth highest cancer killer in the United States among both men and women[1]. Although it accounts for only 2.5% of new cases, pancreatic cancer is responsible for 6% of cancer deaths each year[2]. Less than 5% of patients with the condition survive longer than five years[1]. In three clinical trials in which patients with locally advanced (Stage II, III, or IV (TNM classification of malignant tumours) pancreatic carcinoma were randomized to either observation or combination chemotherapy, the median survival periods averaged 3.5 months in the observation group and 4.5 months in the chemotherapy group[3-6]. The combination of chemotherapy with external beam radiation therapy in randomised clinical trials has yielded a median survival of up to 11.5 months[7-10] (with associated toxicities). More recently, gemcitabine as a single agent showed a median survival of 5.7 months; by contrast, 5-fluorouracil showed a median survival of 4.4 months[11].

The treatment of pancreatic cancer is still problematic for physicians. Only 15% of patients present with resectable tumours, and systemic chemotherapy is of limited effectiveness. In order to achieve higher local drug concentrations in the tumour without causing the side-effects of a comparable level of systemic treatment, regional chemotherapy has been introduced as an alternative treatment and reviewed[12]. Several techniques have been developed over recent years, these include: celiac axis infusion (CAI), CAI with microspheres or haemofiltration, aortic stop flow (ASF) and isolated hypoxic perfusion (IHP). Whilst several authors have reported improved response rates and a prolongation of median survival time, these results have not been confirmed by others. In addition, the incidence of side-effects and the rate of technical complications have been reported to be high during regional chemotherapy.

Endoscopic Ultrasound (EUS) is a medical procedure in which endoscopically directed ultrasound is used to obtain images of the internal organs or tumours in the chest and abdomen. It can be used to guide the injection of drugs or materials into or around the located tumours. It has been shown that EUS alone (94%) is more sensitive than CT scan (69%) and magnetic resonance imaging (83%) for detecting pancreatic lesions, especially when they are smaller than 3 cm[13]. EUS guided injection therapy has been used to deliver chemotherapeutic agents, immune modulators, gene treatment and radionuclide agents in a small number of patients as a way of treating tumours. A phase 1 trial in 2000, delivered a cyto-implant into a pancreatic tumour using EUS guided fine needle injection (FNI) and showed an overall median survival of 13.2 months with a range of 4.2 to >36 months[15]. EUS has been used to guide the implantation of an intratumoural lactic polymer for sustained delivery of 5-fluorouracil to the pancreas. The study demonstrated the technical feasibility of EUS-guided implantation of chemotherapeutic polymers in the canine pancreas[22].

Cystic tumours of the pancreas are frequently detected and encompass a wide pathologic spectrum, ranging from benign to malignant. A substantial proportion of cystic tumours cannot be histologically classified, even after extensive diagnostic evaluation, and, therefore, ultimately require surgical resection. Recently, complete resolution of cystic tumours by EUS-guided ethanol lavage was reported in a pilot study. In a further study, the safety, feasibility, and response after EUS-guided ethanol lavage with paclitaxel injection (EUS-EP) for cystic tumours of the pancreas was evaluated[16]. From this study EUS-EP appears to be a safe, feasible, and effective method for treating cystic tumours of the pancreas.

Intratumoural injection of microspheres has been known for some years and was thought to be problematic. Mattsson *et al*[14] injected micron carbonized microspheres in two transplantable rat tumours, a hepatoma transplanted to the dorsum of one hindpaw and an adenocarcinoma transplanted into the liver. Microspheres were found to be unevenly distributed and often in aggregates that seemed to fill intratumoural vessels. This finding partly explained the increased vascular resistance in tumours after microsphere injection and was thought to make the technique with repeated injections of microspheres disputable[17]. Later studies with albumin microspheres containing adriamycin showed a significant tumour response in a sarcoma rat model compared to intratumoural injection of free drug[18]. The efficacy and toxicity of intratumoural free mitoxantrone or mitoxantrone-loaded albumin microspheres were evaluated in another study using a murine breast cancer model[19]. In the same model, a combination of these two therapies was also evaluated. Results indicated that intratumoural mitoxantrone, especially in microsphere preparations, significantly improved survival and decreased systemic toxicity. Other studies evaluated the ability of injectable, biodegradable microspheres releasing carboplatin, doxorubicin, or 5-fluorouracil to suppress the growth of solid tumours implanted subcutaneously or intramuscularly[20]. 7 to 10 days after implantation of MATB-III cells, rats received systemic chemotherapy, intratumoural bolus chemotherapy, or injections of chemotherapeutic microspheres into the tumour centre or multiple sites along the outer perimeter of the tumour. A single treatment with carboplatin, doxorubicin, or 5-fluorouracil microspheres along the perimeter of the tumours produced a significant, dose-related suppression in tumour growth, relative to injections directly

into the tumour centre. Polypharmacy, accomplished by blending doxorubicin- and 5-fluorouracil-loaded microspheres and injecting them into the tumours was even more efficacious than sustained delivery of either drug alone.

Liu *et al* investigated the effect of delivering doxorubicin and the chemosensitizing agent verapamil from microspheres in a murine breast sarcoma model[21]. Initial *in-vitro* studies confirmed the ability of verapamil to enhance the accumulation of both doxorubicin and [(99mTc)]sestamibi, also a P-glycoprotein substrate, in EMT6 murine breast sarcoma cells and a doxorubicin-selected multidrug-resistant variant, EMT6/AR1.0. There was a modest (up to 34%) delay of tumour growth compared with groups receiving no treatment or blank microspheres. Controlled-release microsphere formulations of anticancer agents administered intratumourally were an efficient way to deliver high drug doses to the tumour with little systemic toxicity.

Further relevant prior art includes WO 2006027567.

There remains a desire to provide improved compositions for the treatment of pancreatic tumours.

<u>Summary of Invention</u>

In accordance with a first aspect of the invention there is provided a composition comprising microspheres which comprise a water-insoluble, water-swellable polymer and associated with the polymer, in releasable form, a chemotherapeutic agent, for use in the treatment of a pancreatic tumour or cyst, wherein the microspheres, when equilibrated in water at 37°C, comprise at least 40 wt% water based on weight of polymer plus water, and wherein the polymer is anionically charged at pH7, and the chemotherapeutic agent is cationically charged and electrostatically associated with the polymer.

In accordance with a second aspect of the invention there is provided a method of treatment of a pancreatic tumour or cyst comprising administering to the human or animal body a composition according to the first aspect of the invention, wherein in the treatment the chemotherapeutic agent is released from the microspheres.

In accordance with a third aspect of the invention there is provided a composition, according to claim 1, comprising a chemotherapeutic agent of general formula I for use in the treatment of a pancreatic tumour or cyst, wherein the agent is directly injected into the tumour or cyst or around its perimeter

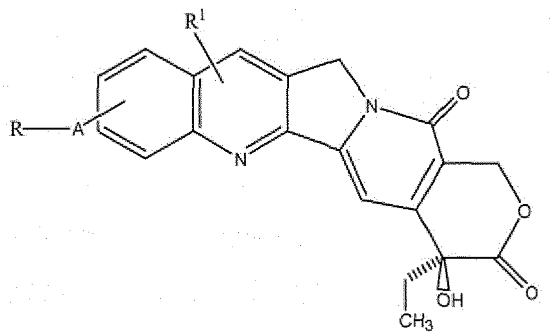

I

in which $R^1$ is selected from H, halogen, hydroxyl and lower ($C_{1-6}$) alkyl, optionally substituted by a hydroxyl, amine, alkoxy, halogen, acyl and acyloxy groups;

A is C(O)O or $CH_2$; and

R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form an optionally substituted heterocyclic ring which may be interrupted by -O-, -S- or $>NR^4$ in which $R^4$ is a hydrogen atom, a substituted or unsubstituted $C_{1-4}$ alkyl group or a substituted or unsubstituted phenyl group;

and wherein the grouping -A-R is bonded to a carbon atom located in any of the positions in the A ring of the camptothecin compound and $R^1$ is substituted in the A or B ring, including salts thereof.

In accordance with a fourth aspect of the invention there is provided a method of treatment of a pancreatic tumour or cyst comprising injecting into the tumour or cyst or around its perimeter a composition according to the third aspect of the invention.

In accordance with a fifth aspect of the invention there is provided a composition, according to claim 1, comprising a polymeric matrix comprising a chemotherapeutic agent of general formula I

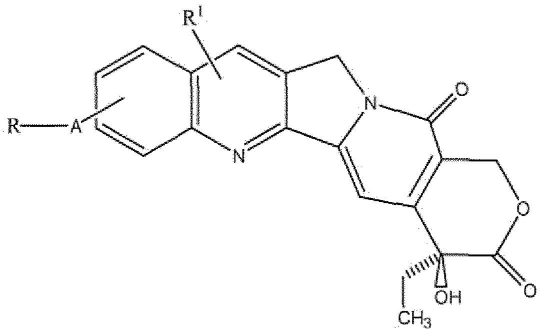

I

in which $R^1$ is selected from H, halogen, hydroxyl and lower ($C_{1-6}$) alkyl, optionally substituted by a hydroxyl, amine, alkoxy, halogen, acyl and acyloxy groups;

A is C(O)O or $CH_2$; and

R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form an optionally substituted heterocyclic ring which may be interrupted by -O-, -S- or >$NR^4$ in which $R^4$ is a hydrogen atom, a substituted or unsubstituted alkyl group or a substituted or unsubstituted phenyl group;

and wherein the grouping -A-R is bonded to a carbon atom located in any of the positions in the A ring of the camptothecin compound and $R^1$ is substituted in the A or B ring, including salts thereof;

for use in the treatment of a pancreatic tumour or cyst.

In accordance with a sixth aspect of the invention there is provided a method of treatment of a pancreatic tumour or cyst comprising injecting into the tumour or cyst or around its perimeter a composition according to the fifth aspect of the invention.

Treatment of pancreatic cancer is extremely difficult since the disease may have already directly invaded critical structures, often displays resistance to chemotherapy and radiotherapy, or has metastasized in most patients by the time of diagnosis. Management options consist of surgical intervention, radiation, chemotherapy, and palliative care measures, such as pain and nutritional management. Surgery is the only option considered curative but this is possible in just 15 to 20% of patients with small tumours confined to the pancreas. The surgical option itself is complex, as the pancreas is surrounded by other organs making access difficult; moreover, the organ is very delicate and must be handled with great care.

The present invention provides local delivery of chemotherapeutic agents from a drug-eluting system. The invention allows high levels of drug to be delivered locally at the tumour or cyst site with significant reductions in systemic toxicity.

Endoscopic ultra-sound guided therapy may be used in combination with the invention. This provides a safe and precise method of accessing the pancreas. It can be used to guide the delivery of chemotherapeutic beads to treat pancreatic lesions, with the potential to enjoy the benefits of high local drug concentrations in the lesion (offering the possibility of overcoming resistance mechanisms) over sustained periods.

<u>Detailed Description of Invention</u>

<u>Microspheres</u>

Microspheres for use in this invention have been described in the Applicant's previous applications - see for instance, WO04/071495, WO06/027567 and WO08/128580 (in particular see the Reference Examples). However, they have never been described for use in the treatment of a pancreatic tumour by direct injection before.

In the first and second aspects of the invention, the water swellable polymer is anionically charged at pH7 and the chemotherapeutic agent is cationically charged, and electrostatically associated with the polymer. These form preferred embodiments of all other aspects of the invention.

The deformable nature of the polymer which comprises the microspheres enables the microspheres to be deliverable by needles for direct injection into the desired site. The microspheres may be degradable, non-degradable, or even combined with a radiosensitizing agent to enable the microsphere treatment to be combined with radiotherapy.

The polymer is a water-insoluble material. Although it may be biodegradable, so that active may be released substantially by erosion from the surface of the polymer matrix, preferably the polymer is substantially biostable (i.e. non-biodegradable).

The polymer is water-swellable. Water-swellable polymer in the present invention has a equilibrium water content, when swollen in water at 37°C, measured by gravimetric analysis of at least 40 wt%, preferably in the range of 40 to 99 wt%, most preferably 75 to 95%.

In a preferred embodiment of the invention, the composition is in the form of a suspension of microspheres of water-swollen water-insoluble polymer in a liquid carrier. Typically, the microspheres have sizes when equilibrated in water at 37°C, in the range 1-1000 μm, more preferably in the range 50 to 500 μm, most preferably in the range 100-300 μm. Preferably the microspheres are substantially spherical in shape. The diameter is preferably determined by measurement of the microsphere size prior to loading with the cationically charged chemotherapeutic agent. Although the microspheres are preferably substantially spherical, they may be spheroidal or even less regular in shape. The diameter of a non-spherical microsphere is its largest diameter.

Generally the polymer is covalently crosslinked, although it may be appropriate for the polymer to be ionically crosslinked, at least in part.

Polymers which are derived from natural sources, such as albumin, alginate, gelatin, starch, chitosan or collagen may be used in one embodiment of the invention. Alginate is particularly preferred. Alginate microspheres are typically prepared from super-pure alginate with either High G or High M content by extrusion of an alginate solution of specific concentration into a gelling bath of metal (e.g. calcium or barium) ions. Methods for making alginate microspheres suitable for use in the invention are described in WO08/128580 in particular Examples 8-11.

In another embodiment the polymer is formed by polymerising ethylenically unsaturated monomers in the presence of di- or higher-functional crosslinking monomers. The ethylenically unsaturated monomers may include an ionic (including zwitterionic) monomer.

Copolymers of hydroxyethyl methacrylate, acrylic acid and cross-linking monomer, such as ethylene glycol dimethacrylate or methylene bisacrylamide, as used for etafilcon A based contact lenses may be used. Copolymers of N-acryloyl-2-amino-2-hydroxymethyl-propane-1,3-diol and N,N-bisacrylamide may also be used.

Other polymers are cross-linked styrenic polymers e.g. with ionic substituents, of the type used as separation media or as ion exchange media.

Another type of polymer which may be used to form the water-swellable water-insoluble matrix is cross-linked polyvinyl alcohol. The polymer may, for instance, be crosslinked using aldehyde-type crosslinking agents such as glutaraldehyde. For such products, the polyvinyl alcohol (PVA) may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The polyvinyl alcohol may alternatively be a copolymer of vinyl alcohol and an anionic acrylic monomer such as an acrylic acid, of the type used as super-absorbent polymers.

The invention is of particular value where the polymer matrix is formed from a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer including anionic monomer.

The PVA macromer may be formed, for instance, by providing PVA polymer, of a suitable molecular weight such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved named nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula III

$$Y^1BQ^1 \qquad III$$

in which $Y^1$ is selected from

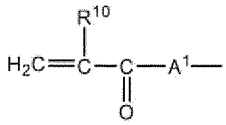

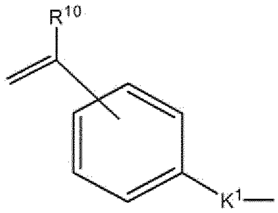

$CH_2=C(R^{10})-CH_2-O-$, $CH_2=C(R^{10})-CH_2\ OC(O)-$, $CH_2=C(R^{10})OC(O)-$, $CH_2=C(R^{10})-O-$, $CH_2=C(R^{10})CH_2OC(O)N(R^{11})-$, $R^{12}OOCCR^{10}=CR^{10}C(O)-O-$, $R^{10}CH=CHC(O)O-$, $R^{10}CH=C(COOR^{12})CH_2-C(O)-O-$,

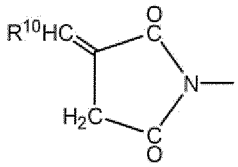

and

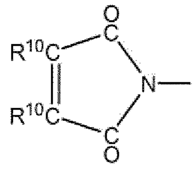

wherein:

$R^{10}$ is hydrogen or a $C_1$-$C_4$ alkyl group;
$R^{11}$ is hydrogen or a $C_1$-$C_4$ alkyl group;
$R^{12}$ is hydrogen or a $C_{1-4}$ alkyl group or $BQ^1$ where B and $Q^1$ are as defined below;
$A^1$ is -O- or-$NR^{11}$-;
$K^1$ is a group $-(CH_2)_rOC(O)-$, $-(CH_2)_rC(O)O-$, $-(CH_2)_rOC(O)O-$,$-(CH_2)_rNR^{13}-$, $-(CH_2)_rNR^{13}C(O)-$, $-(CH_2)_rC(O)NR^{13}-$, $-(CH_2)_rNR^{13}C(O)O-$,$-(CH_2)rOC(O)NR^{13}-$, $-(CH_2)_rNR^{13}C(O)NR^{13}-$ (in which the groups $R^{13}$ are the same or different), $-(CH_2)_rO-$, $-(CH_2)_rSo_3-$, or, optionally in combination with B, a valence bond and r is from 1 to 12 and $R^{13}$ is hydrogen or a $C_1$-$C_4$ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if $Q^1$ or $Y^1$ contains a terminal carbon atom bonded to B a valence bond; and $Q^1$ is an ionic group.

Such a compound including an anionic group $Q^1$ is preferably included.

An anionic group $Q^1$ may be, for instance, a carboxylate, carbonate, sulphonate, sulphate, nitrate, phosphonate or phosphate group. The monomer may be polymerised as the free acid or in salt form. Preferably the $pK_a$ of the conjugate acid is less than 5.

A suitable cationic group $Q^1$ is preferably a group $N^+R^{14}{}_3$, $P^+R^{15}{}_3$ or $S^+R^{15}{}_2$ in which the groups $R^{14}$ are the same or different and are each hydrogen, $C_{1-4}$-alkyl or aryl (preferably phenyl) or two of the groups $R^{14}$ together with the heteroatom to which they are attached form a saturated or unsaturated heterocyclic ring containing from 5 to 7 atoms. The groups $R^{15}$ are each $OR^{14}$ or $R^{14}$. Preferably the cationic group is permanently cationic, that is each $R^{14}$ is other than hydrogen. Preferably a cationic group Q is $N^+R^{14}{}_3$ in which each $R^{14}$ is $C_{1-4}$-alkyl, preferably methyl.

A zwitterionic group $Q^1$ may have an overall charge, for instance by having a divalent centre of anionic charge and monovalent centre of cationic charge or vice-versa or by having two centres of cationic charge and one centre of anionic charge or vice-versa. Preferably, however, the zwitterion has no overall charge and most preferably has a centre of monovalent cationic charge and a centre of monovalent anionic charge.

Examples of zwitterionic groups which may be used as Q in the present invention are disclosed in WO-A-0029481.

Where the ethylenically unsaturated monomer includes zwitterionic monomer, for instance, this may increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxyethyl phosphorylcholine (MPC).

In the monomer of general formula I preferably $Y^1$ is a group $CH_2=CR^{10}COA^1$- in which $R^{10}$ is H or methyl, preferably methyl, and in which $A^1$ is preferably NH. B is preferably an alkanediyl group of 1 to 12, preferably 2 to 6 carbon atoms. Such monomers are acrylic monomers.

There may be included in the ethylenically unsaturated monomer diluent monomer, for instance non-ionic monomer. Such a monomer may be useful to control the $pK_a$ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylarnides, especially such compounds

having alkyl groups with 1 to 12 carbon atoms, hydroxy, and di-hydroxy-substifiuted alkyl(alk) acrylates and -(alk) acrylamides, vinyl lactams, styrene and other aromatic monomers.

In the polymer matrix, the level of anion is preferably in the range 0.1 to 10 meq $g^{-1}$, preferably at least 1.0 meq $g^{-1}$. Preferred anions are derived from strong acids, such as sulphates, sulphonates, phosphates and phosphonates.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

The crosslinked polymer may be formed as such in particulate form, for instance by polymerising droplets of ethylenically unsaturated monomer in a dispersed phase in a continuous immiscible carrier. This is especially suitable for the polyvinyl alcohol macromer-based polymerisation. Examples of suitable water-in-oil polymerisations to produce microspheres having the desired size, when swollen, are known. For instance US 4,224,427 describes processes for forming uniform spherical beads (microspheres) of up to 5 mm in diameter, by dispersing water-soluble monomers into a continuous solvent phase, in a presence of suspending agents. Cross-linking preformed polymerising droplets of a dispersion can also be used. Cross-linking preformed polymer in droplets of a dispersion can also be used. Stabilisers and surfactants may be present to provide control over the size of the dispersed phase particles. After polymerisation, and/or cross-linking, the crosslinked microspheres are recovered by known means, and are washed and optionally sterilised. Preferably the microspheres are swollen in an aqueous liquid, and classified according to their size.

A cationically charged chemotherapeutic agent (hereinafter also referred to as "active" or "drug") is associated with the polymer preferably so as to allow controlled release of the active over a period. This period may be from several minutes to weeks, preferably at least up to a few days, preferably up to 72 hours. The active is electrostatically bonded to the polymer. The presence of anionic groups in the polymer allows control of release of cationically charged active.

In the invention it is important that the drug is not covalently attached to the polymer matrix.

The active may be incorporated into the polymer matrix by a variety of techniques. In one method, the active may be mixed with a precursor of the polymer, for instance a monomer or macromer mixture or a cross-linkable polymer and cross-linker mixture, prior to polymerising or crosslinking. Alternatively, the active may be loaded into the polymer after it has been crosslinked. For instance, particulate dried polymer may be swollen in a solution of active, preferably in water or in an alcohol such as ethanol, optionally with subsequent removal of non-absorbed agent and/or evaporation of solvent. A solution of the active, in an organic solvent such as an alcohol, or, more preferably, in water, may be sprayed onto a moving bed of microspheres, whereby drug is absorbed into the body of the microspheres with simultaneous solvent removal. Most conveniently, we have found that it is possible merely to contact swollen microspheres suspended in a continuous liquid vehicle, such as water, with an aqueous alcoholic solution of drug, over a period, whereby drug becomes absorbed into the body of the microspheres. Techniques to fix the drug in the microspheres may increase loading levels, for instance, precipitation by shifting the pH of the loading suspension to a value at which the active is in a relatively insoluble form. The swelling vehicle may subsequently be removed or, conveniently, may be retained with the microspheres as part of the product. The swollen microspheres may be used in swollen form in the form of a slurry, i.e. without any or much liquid outside the swollen microspheres. Alternatively, the suspension of microspheres can be removed from any remaining drug loading solution and the microspheres dried by any of the classical techniques employed to dry pharmaceutical-based products. This could include, but is not limited to, air drying at room or elevated temperatures or under reduced pressure or vacuum; classical freeze-drying; atmospheric pressure-freeze drying; solution enhanced dispersion of supercritical fluids (SEDS). Alternatively the drug-loaded microspheres may be dehydrated using an organic solvent to replace water in a series of steps, followed by evaporation of the more volatile organic solvent. A solvent should be selected which is a non-solvent for the drug.

In brief, a typical classical freeze-drying process might proceed as follows: the sample is aliquoted into partially stoppered glass vials, which are placed on a cooled, temperature controlled shelf within the freeze dryer. The shelf temperature is reduced and the sample is frozen to a uniform, defined temperature. After complete freezing, the pressure in the dryer is lowered to a defined pressure to initiate primary drying. During the primary drying, water vapour is progressively removed from the frozen mass by sublimation whilst the shelf temperature is controlled at a constant, low temperature. Secondary drying is initiated by increasing the shelf temperature and reducing the chamber pressure further so that water absorbed to the semi-dried mass can be removed until the residual water content decreases to the desired level. The vials can be sealed, in situ, under a protective atmosphere if required.

Atmospheric pressure freeze-drying is accomplished by rapidly circulating very dry air over a frozen product. In comparison with the classical freeze-drying process, freeze-drying without a vacuum has a number of advantages. The circulating dry gas provides improved heat and mass transfer from the frozen sample, in the same way as washing dries quicker on a windy day. Most work in this area is concerned with food production, and it has been observed that there is an increased retention of volatile aromatic compounds, the potential benefits of this to the drying of biologicals is yet to be determined. Of particular interest is the fact that by using atmospheric spray-drying processes, instead of a cake, a fine, free-flowing powder is obtained. Particles can be obtained which have submicron diameters, this is ten-fold smaller than can be generally obtained by milling. The particulate nature, with its high surface area results in an easily rehydratable product, currently the fine control over microsphere size required for inhalable and transdermal applications is not possible, however there is potential in this area.

Although the composition, according to claim 1, may be made up from polymer and chemotherapeutic agent (active) immediately before administration, it is preferred that the composition is preformed. Dried polymer-active microspheres may be hydrated immediately before use. Alternatively the composition which is supplied may be fully compounded and preferably comprises polymer microspheres with absorbed or adsorbed active compound and imbibed water e.g. physiological saline and extra-particulate liquid, for instance saline.

In the third aspect of this invention, a composition, according to claim 1, comprising a chemotherapeutic agent of general formula I for the treatment of a pancreatic tumour by direct injection is provided. Preferably, the agent is in a polymeric matrix, as in the fifth aspect of the invention. The polymer in the polymer matrix is preferably a water-insoluble material. Although it may be biodegradable, so that drug may be released substantially by erosion of polymer matrix to release drug from the surface, preferably the polymer is substantially biostable (i.e. non-biodegradable). Preferably, the polymer matrix is in the form of microspheres, as in the first aspect of the invention.

The polymer is water-swellable. Water-swellable polymer useful in the invention preferably has an equilibrium water content, when swollen in water at 37°C, measured by gravimetric analysis, in the range of 40 to 99 wt%, preferably 75 to 95%.

Generally the polymer is covalently crosslinked, although it may be appropriate for the polymer to be ionically crosslinked, at least in part, or hydrophobically crosslinked. In some embodiments it may be suitable to use polymers which are derived from natural sources, such as albumin, alginate, gelatin, starch, chitosan or collagen, all of which have been used as embolic agents. In other embodiments the polymer is substantially free of naturally occurring polymer or derivatives. It is preferably formed by polymerising ethylenically unsaturated monomers in the presence of di- or higher-functional crosslinking monomers. The ethylenically unsaturated monomers may include an ionic (including zwitterionic) monomer.

The level of active in the composition which is administered is preferably in the range 0.1 to 500 mg per ml composition, preferably 10 to 100 mg per ml. Preferably the treatment is repeated one to five times and for each dose the amount of active administered is in the range 0.1 to 100 mg per ml, preferably 10 to 100 mg per ml. The amount of composition administered in a normal treatment is in the range 1 to 6 ml. The total amount of active administered per dose is preferably in the range 10 to 1000 mg, more preferably 50 to 250 mg. Based on the release data as shown in the Examples below, the inventors believe this will give therapeutically effective concentrations in the tumour and that significant levels of intracellular delivery should take place whereby a therapeutic effect will be achieved. The adverse side effects of active administration should be avoided.

Methods for incorporating such drugs into microspheres are explained in detail in WO2007/09089

Chemotherapeutic Agent

In the first aspect of the invention, a cationically-charged chemotherapeutic agent is used.

The chemotherapeutic agent (active) used in the first aspect of this invention is cationically charged and therapeutically active against pancreatic tumours. The microspheres, as defined above, release the cationically charged chemotherapeutic agent via an ion-exchange mechanism, and thereby act as a depot for the controlled delivery of the cationically charged chemotherapeutic agent to a pancreatic tumour.

The chemotherapeutic agent may be a prodrug, that is, a compound which is activated *in vivo* to form the active. The chemotherapeutic agent may be a radiosensitiser. A radiosensitiser is a compound that increases the radiosensitivity of tissues, particularly tumour cells.

Suitable cationically charged compounds are cationic camptothecin derivatives, such as irinotecan, as described in WO2006027567.

In one embodiment of the invention, two or more chemotherapeutic agents are used in the treatment of the pancreatic tumour. In this case, the combination of drugs tested may involve separate particles, each loaded with one of the drugs or each particle may be jointly loaded with both the drugs. Methods for the co-loading of microspheres are given in the Applicant's copending application PCT/GB2008/050722.

One of the agents is cationically charged, but the second agent need not be. One of the agents should be cytotoxic, and the other should have activity complementary to the cytotoxic compound in tumour treatment. Such combinations are described in more detail in PCT/GB2008/050722.

One class of cytotoxic or cytostatic compounds used in the combination as the second agent comprises rapamycin and rapamycin analogues, which target mTOR. Such compounds include sirolimus, temsirolimus, everolimus, biolimus, ABT-578 and AP23573. Any of the compounds encompassed within the scope of rapamycin analogues described in WO-A-2003022807, may be used as the rapamycin analogue.

The chemotherapeutic agent used in the combination may be hydrophobic. The present invention has been

found to be of utility for formulating combinations including drugs having anti-tumour properties and low solubility in water, with higher solubility in a water-miscible organic solvent. Combinations including rapamycin and derivatives having solubility in water at room temperature less than 10g/l are used in the invention. All of these compounds have a solubility ratio in a water-miscible solvent to water at room temperature of at least 10:1, preferably at least 100:1, up to as much as 106:1 or even more, for instance more than 103:1.

Verapamil and PGP inhibitors are often used in combination therapy in this invention together with cationically charged chemotherapeutic agents.

Where the agents include a camptothecin derivative, it is particularly effective to use a derivative having cationic substituents, such as compounds of the general formula I as described above. Preferably, the grouping -A-R is bonded to a carbon atom located in any of the 9, 10 or 11 positions in the A ring of the camptothecin compound.

In one embodiment the camptothecin derivatives have the formula IA

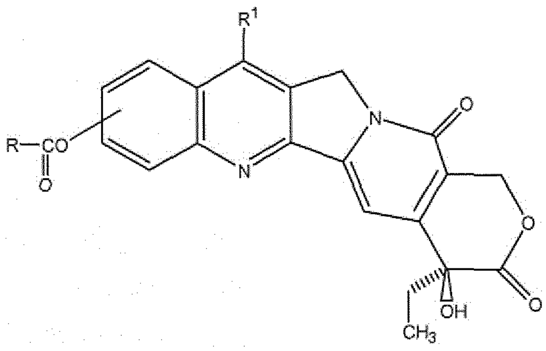

IA

in which $R^1$ is H, lower ($C_{1-6}$) alkyl, optionally substituted by a hydroxyl, amine, alkoxy, halogen, acyl or acyloxy group or halogen; and

R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form an optionally substituted heterocyclic ring which may be interrupted by -O-, -S- or $>NR^4$ in which $R^4$ is a hydrogen atom, a substituted or unsubstituted $C_{1-4}$ alkyl group or a substituted or unsubstituted phenyl group;

and wherein the grouping -O-CO-R is bonded to a carbon atom located in any of the 9, 10 or 11 positions in the A ring of the camptothecin compound, including salts thereof.

It is preferred for the grouping -C-CO-R to be joined at the 10 position.

$R^1$ is preferably $C_{1-4}$ alkyl, most preferably ethyl, and m is preferably 1.

A halogen atom R is, for instance, F, Cl, Br or I, preferably F or Cl. $R^1$ to $R^4$ may be methyl, ethyl, propyl, isopropyl, in-butyl, isobutyl and t-butyl, preferably methyl.

Substituents in R and $R^1$ are preferably selected from halogen atoms, hydroxy, $C_{1-4}$ alkoxy, phenoxy, $COOR^6$, $SO_3R^6$ and $PO_3(R^6)_2$, aryl,

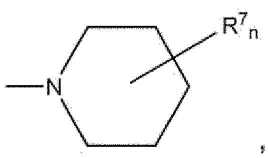

,

$NR^8R^9$ and $CONR^8R^9$, $QAOR^5$, $QANR^8R^9$ and $QAQR^5$ in which $R^5$ is $C_{1-4}$ alkyl or aryl; $R^6$ is hydrogen, halogen $C_{1-4}$ alkyl or $C_{1-4}$ alkoxy; $R^7$ is hydrogen, halogen or $C_{1-4}$ alkyl; $R^8$ and $R^9$ are the same or different and each is H, or $C_{1-4}$ alkyl or $R^3$ and $R^9$ together represent $C_{3-6}$, alkanediyl;

Q is OCO, or -COO- and A is $C_{2-4}$ alkanediyl.

Preferably R is $NR^3$ $R^3$ where $R^2$ and $R^3$ together with the nitrogen atom form a 5 or 6 membered ring, preferably a saturated ring, with optional substituents. A substituent is preferably $-NR^8R^9$. In such a substituent $R^8$ and $R^9$ preferably together are $C_{4-5}$ alkanediyl. Such groups are basic and tend to be cationically charged at pH7. Most preferably R is

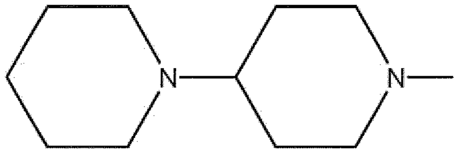

The composition may comprise an imaging agent such as a conventional radiopaque agent, or a dye. The composition which is introduced may also be admixed with other therapeutic actives, or may be introduced separately but in combination with other actives.

The composition used to treat the pancreatic tumour is typically an aqueous suspension of swollen microspheres containing absorbed active. It is desirable to mix the suspension prior to delivery with any of the additional agents discussed above. Alternatively or additionally the microspheres may be pre-loaded with any of these additional agents.

Treatment

The invention may be used to treat pancreatic tumours or cysts. The tumour may be cancerous or benign. A cyst is a collection of fluid within the head, body or tail of the pancreas. Some cysts are benign. However, others are cancerous or precancerous, and the present invention may be used to treat both.

To treat the tumour or cyst, the composition, according to claim 1, is typically injected into the vicinity of the tumour or cyst. By vicinity, is meant injection directly into the tumour or cyst, or injection at the periphery/on the perimeter of the tumour or cyst. The injection site may alternatively lie outside the perimeter of the tumour or cyst, but should be in sufficiently close proximity to it such that the chemotherapeutic agent is able to influence the growth of the tumour or cyst.

Normally the composition, according to claim 1, will be injected directly into the pancreas. However, in some circumstances, the composition may be injected outside the pancreas but sufficiently close to it such that the chemotherapeutic agent is able to interfere with tumour or cyst growth.

Preferably, to treat a pancreatic tumour or cyst, the composition is injected into the pancreas, typically directly into the pancreas using a needle. Typically, the composition is injected around the peripheral margins of the tumour or cyst.

Injection of the composition, according to claim 1, in this manner allows the chemotherapeutic agent to be released in a controlled manner at its target location. This means that smaller doses can be used, which limits the problems associated with systemic toxicity.

Endoscopic ultrasound (EUS) can be used in the treatment to visualise the tumour and guide the injection of chemotherapeutic agent into the tumour. EUS normally involves the insertion of an echo endoscope into the oesophagus of a human or animal subject, and passing this through into the proximal stomach. The composition, according to claim 1, is then typically inserted into the lumen of a needle with an appropriate Gauge, and this is attached to the endoscopic apparatus. The needle may be pushed through the gastric wall and into the pancreas under continuous sonographic guidance. The composition is then pushed out of the needle and implanted into the tissue. The apparatus is then removed from the body.

When injected into tissues, the microspheres have a tendency to be pushed out by the intratissular pressure. To overcome this problem, the composition which is introduced may additionally comprise a viscosity modifier. The viscosity modifier should increase the viscosity of the composition. The viscosity modifier may be, for instance, alginate, a carboxycellulosic or a biocompatible polymer such as PVP. When alginate is used as the viscosity modifier, the alginate advantageously forms a gel once injected by cross-linking with calcium ions which have diffused from surrounding tissue.

Preferably ultra high purity, high molecular weight alginate is used as a viscosity modifier. The injection of foreign materials into the pancreas can cause inflammation, leading to pancreatitis. Alginate shows good biocompatibility and therefore limits the inflammatory response.

Typically, the alginate has a molecular weight of greater than 300 kDa, more typically greater than 500 kDa, and most typically more than 800 kDa. Phycomer E01 is a suitable example of an alginate which can act as a viscosity modifier.

Subjects treated in this invention are generally mammalian, and are preferably humans.

The invention is further illustrated in the following Examples. Some of the results are shown in the accompanying Figures, described in more detail in the Examples, but briefly described as follows:

Figure 1: PVA hydrogel beads loaded with topotecan at 25mg/mL;

Figure 2: Topotecan and irinotecan elution from 300-500 mm PVA-hydrogel beads into 200 mL PBS;

Figure 3: Topotecan and irinotecan elution as percentage of total loading - 300-500 mm PVA-hydrogel beads, 200 mL PBS, ambient temperature;

Figure 4: PSN1 cell viability after exposure to various concentrations of irinotecan and topotecan for 24hrs. (n=7; +/- SD);

Figure 5: PSN1 cell viability after exposure to various concentrations of irinotecan and topotecan for 48hrs. (n=7; +/- SD);

Figure 6: PSN1 cell viability after exposure to various concentrations of irinotecan and topotecan for 72hrs. (n=7; +/- SD);

Figure 7: Tumour size change of nude mouse with PSN1 tumour xenograft over 25 days after direct injection of i) irinotecan beads in alginate and ii) alginate only. (mean +/- SD, n=3);

Figure 8: Mean weight change of nude mouse with PSN1 tumour xenograft over 25 days after direct injection of i) irinotecan beads in alginate and ii) alginate only. (mean +/- SD, n=3);

Figure 9: PSN1 cell viability after exposure to 1, 2 or 3 irinotecan loaded beads for 24, 48 and 72 hours. (mean +/- SD, n=7);

Figure 10: PSN1 cell viability after exposure to 1 rapamycin, 1 irinotecan and a combination of 1 rapamycin and 1 irinotecan bead for 24, 48 and 72 hours. (mean +/- SD, n=7);

Figure 11: Tumour size change of nude mouse with PSN1 tumour xenograft over 25 days after direct injection of i) irinotecan beads in alginate and ii) alginate only. (mean +/- SD, n=3); and

Figure 12: Mean weight change of nude mouse with PSN1 tumour xenograft over 25 days after direct injection of i) irinotecan beads in alginate and ii) alginate only. (mean +/- SD, n=3)

Figure 13: Tumour volume change for PSN1 subcutaneous transplantation in female nude mice when treated with A) irinotecan (- • 3.3 mg; - 6.6mg) B) topotecan (- • • 0.2 mg; • • • 0.4 mg);

Figure 14: Mean weight change of nude mouse with PSN1 tumour xenograft 60 days after direct injection of irinotecan or topotecan beads in alginate (mean +/-SD, n=3);

Figure 15: CT scan after injection in Example 9; and

Figure 16: Pathological grading of inflammation in pancreatic tissue surrounding injection.

**Reference Example: Outline Method for the Preparation of Microspheres**

The microspheres are synthesised by the method described in WO2004/071495 as either the "low AMPS" or "high AMPS" product. The "high AMPS" product is used in the following Examples. Briefly, an aqueous mixture of polyvinyl alcohol macromer having acetal-linked ethylenically unsaturated groups and 2-acrylamido-2-methyl-propane sulphonate in a weight ratio of about 1:1 is suspended in a continuous phase of butyl acetate containing cellulose acetate butyrate stabiliser with agitator and is radically polymerised using redox initiation to form beads, which are washed, dyed and sieved into size fractions including the 100-300μm fraction used in subsequent Examples. The equilibrium water content of the microspheres is 94 to 95% by weight.

**Comparative Example 1: Preparation of Irinotecan-loaded PVA-hydrogel Beads.**

Irinotecan-loaded sulphonate-modified PVA hydrogel microspheres were prepared as detailed in WO2006/02756 (see Example 1 for loading and elution of Irinotecan from embolisation beads). Drug-loaded beads were lyophilised (see Example 5 of WO2006/027567) to remove water and sterilised using gamma irradiation.

**Comparative Example 2: Preparation of Topotecan-loaded PVA-hydrogel Beads.**

Topotecan-loaded sulphonate-modified PVA hydrogel microspheres were prepared by taking 22.73 mg of topotecan (yellow powder Dabur Pharma Ltd) and dissolving it into 5 mL water to make a solution of 4.55 mg/mL. 4.39 mL of this solution was mixed with 1 mL of PVA hydrogel bead slurry (500-700 μm size range), and the solution turned from yellow to colourless and the blue beads turned green within an hour. Figure 1 shows an image of the loaded PVA hydrogel beads which are green in colour. According to the depletion measurement of drug residue in loading solution, the estimated drug loading is 19.8 mg, and loading efficiency is 99.2%. The size of loaded beads is 492 ± 42 μm, which is decreased compared to -600 μm unloaded PVA hydrogel beads in 500-700 μm range.

**Comparative Example 3: Comparison of Elution of Irinotecan and Topotecan PVA-hydrogel Beads.**

1 mL of PVA-hydrogel beads were roller-mixed with 3 mL 15.46 mg/mL topotecan solution for 3 hr. The loading was measured as 38.7 mg/mL beads (average of two replicates) by depletion method using PE Lamda 25 UV at 384 nm. The loaded beads were separated from loading solution and roller-mixed with PBS 200 mL at ambient temperature. At certain time points, aliquots of solution were taken and diluted for UV measurement at 384 nm for concentration determination. The elution profile are shown in Figures 2 and 3, and compared with that of Irinotecan loaded beads prepared using a similar method as outlined in Example 1.

**Comparative Example 4: Irinotecan and topotecan cytotoxicity on PSN1 (Human Pancreatic Carcinoma) Cell Line.**

20,000 PSN1 cells were seeded in 96 well plates and left to adhere to the well surface overnight. The drug solutions underwent serial dilutions from 1000μg/ml to $1x10^{-8}$ μg/ml in cell culture media. The media was removed from the cells and 200μl of each concentration was added to the cells (n=7); a blank control had 200μl media added (n=12). After 24, 48 and 72hrs, the cells were washed (x3) with PBS and 100μl media was added. The MTS assay was conducted by adding 20μl MTS solution to each well and incubating (37°C, 5% $CO_2$) for 2hrs. The absorbance of each well at 490nm was determined using a Biotek plate reader. Cell viability was calculated as a percentage of the control group (all samples had the media & MTS solution blank absorbance value subtracted).

Both irinotecan and topotecan reduced the viability of the PSN1 cells. Irinotecan required a higher dose at all time points that topotecan to achieve a 50% loss of cell viability (Table 1).

Table 1: Estimated IC50 value of topotecan and irinotecan at 24, 48 and 72 hrs.

| | **IC50 conc. (μg/ml)** | | |
|---|---|---|---|
| | **24** | **48** | **72** |
| **Topotecan** | 600 | 0.6 | 0.1 |
| **Irinotecan** | 100 | 35 | 30 |

After 24hrs exposure topotecan began to show a reduction in cell viability at 1 μg/ml, whereas irinotecan did not show a reduction until the concentration was 100 μg/ml (Figure 4). At 48 hours topotecan started showing activity ( ≈ 80% cell viability) at 0.1 μg/ml, whilst irinotecan showed a reduction in cell viability at 10 μg/ml (Figure 5). After 72 hours irinotecan began to show an effect around 50 μg/ml (Figure 6). Topotecan in contrast showed activity as low as 1 $x10^{-7}$ μg/ml; although a reduction in cell viability below 80% was not achieved until the cells were exposed a concentration of 0.01 μg/ml (Figure 6). Topotecan was more potent than irinotecan in reducing PSN1 cell viability. At lower concentration (<1 μg/ml) irinotecan showed no detrimental effect in cell viability, even after 72 hours. Topotecan even demonstrated cytotoxic effects at 72 hours at several concentrations that had shown no effect after 24 and 48 hours.

**Comparative Example 5: *In vivo* Direct Injection of Irinotecan Loaded Beads into Human Pancreatic Carcinoma Xenograft on Nude Mice.**

*Pre-treatment:* $10^7$ PSN-1 cells (cell line) were prepared from an *in vitro* passage for subcutaneous transplantation. Mice were randomised to the scheduled treatment.

*Anaesthesia:* None

*Clinical Procedure:* When the tumours became palpable (2-3mm) the irinotecan beads / alginate mixture was injected close to the subcutaneous growing tumour (this occurred 5 days after tumour cell injection).

Prior to injection the lyophilised sterilised irinotecan beads were hydrated by the addition of 3ml alginate solution (AS-021). The samples were mixed on a vortex mixer to ensure an even distribution of beads within the alginate. The total volume delivered was 100μl (alginate solution and beads) per mouse. The irinotecan beads contained 3.2 mg of irinotecan. The body weight of each mouse was determined twice per week as a measure of toxicity.

The mice injected with irinotecan loaded beads showed efficacy in reducing tumour volumes at all time points when compared to the control group (Figure 7). The tumour size continued to decrease until day 14 when the tumour volume began to increase (perhaps due to the entire drug being eluted from the beads). The body weight of the irinotecan treated mice increased from 20g to 25g; this can be used as an indication no toxicity (Figure 8). A decrease in body weight occurred from day 21 to 25 (an indication of toxicity); this is likely to be due to tumour growth since no weight loss occurred 11 days after treatment with irinotecan. The irinotecan treated mice were euthanized after the 25 day time point because the tumour was greater than 10% of the total weight of the mouse.

**Example 6: Irinotecan and Rapamycin Drug Eluting Beads Cytotoxicity *in vitro.***

PSN1 cells were diluted to a concentration of 100,000 cells $ml^{-1}$ in the appropriate cell media. Using an eight channel multiwell pipet, 200 μl of cell solution was added to each well of a sterile flat bottomed 96 well plates (Cellstar®, 655180) were seeded with 20,000 cells in 200 media and left in an incubator (37°C, 5% $CO_2$) 20 hours. After 20 hrs the cell culture media was removed and replaced with fresh media.

Fresh media (200 μl) was added to the wells. Pre-loaded irinotecan (30mg/ml) or rapamycin (20mg/ml) microspheres or were carefully counted from pools of the microspheres in sterile $H_2O$ into the wells using a pipette set to 5 μl. The rapamycin beads were prepared as detailed in Example 2 of WO2007/090897. The samples analysed were: 1 - 3 irinotecan beads were per well (see Figure 9). For the combination work, the well contained 1 rapamycin and 1 irinotecan bead together and this was compared against irinotecan and rapamycin beads on their own. The action of pipetting microspheres did add volume to the media in the wells so the corresponding amount was removed afterwards. For example, if three microspheres were pipetted into one well, 15 μl was removed at the end. Once the microsphere had been added the wells were incubated for 24, 48 or 72hrs as required.

At the designated time point, the media (with or without microspheres) was removed and the cells were washed three times with 200 μl PBS to remove any left over drug or microspheres. Cell culture media (100 μl) was added to the wells followed by 20 μl MTS solution (Promega, UK). The cells were incubated (37°C, 5% $CO_2$) for 2 hours and gently agitated before the absorbance at 490nm was recorded using a Biotek plate reader. Media only and untreated cells absorbances were taken as controls.

Cell viability was calculated using the following equation:

$$\% \text{ cell viability} = \left( \frac{(\text{sample}_{\text{abs 490nm}} - \text{media}_{\text{abs 490nm}})}{(\text{control}_{\text{abs 490nm}} - \text{media}_{\text{abs 490nm}})} \right) \times 100$$

Equation 1: *Cell viability calculation*

Irinotecan loaded beads decreased PSN1 cell viability after eluting for 48 and 72 hours (Figure 9). The number of beads per well affected the amount of viable cells, with more beads leading to increased cell death. No cell toxicity was seen after 24 hours; instead cell numbers were increased by 5-30% (depending on the number of beads present). The cell proliferation after 24 hours could be due to a stress induced cell survival mechanism.

When used in combination, irinotecan and rapamycin showed a significant (t-test $p<0.01$) decrease in cell viability compared to either drug alone after 48 or 72 hrs (Figure 10).

**Comparative Example 7: *In vivo* Direct Infection of Doxorubicin Loaded Beads into Human Pancreatic Carcinoma Xenograft on Nude Mice.**

*Pre-treatmenf:* $10^7$ PSN-1 cells (cell line) were prepared from an *in vitro* passage for subcutaneous transplantation. Mice were randomised to the scheduled treatment.

*Anaesthesia:* None

*Clinical Procedure:* When the tumours became palpable (2-3mm) the doxorubicin beads / alginate mixture was injected close to the subcutaneous growing tumour (this occurred 5 days after tumour cell injection).

Prior to injection the lyophilised sterilised doxorubicin PVA-hydrogel beads (Dox PVA) were hydrated by the addition of 3ml alginate solution (AS-021). See Example 2 of WO2004/071495 for the loading of doxorubicin into microspheres, in combination with Example 5 for high AMPS loading. The samples were mixed on a vortex mixer to ensure an even distribution of beads within the alginate. The total volume delivered was 100μl (alginate solution and beads) per mouse. The doxorubicin beads contained 2.5 mg of doxorubicin. The body weight of each mouse was determined twice per week as a measure of toxicity. Alginate beads were also loaded with doxorubicin (Dox-Alg) and diluted to attain the same volume of beads as for the PVA-hydrogel example. The beads were prepared in accordance with the procedure set out in Examples 8-11 of WO08/128580 These beads were injected in a slurry of DOX-loaded alginate beads together with unloaded alginate beads. The unloaded beads were added to the mixture to ensure that the dilution was the same as the DOX-loaded DC bead in alginate solution.

The mice injected with doxorubicin loaded beads of both types showed efficacy in reducing tumour volumes at all time points when compared to the control group (Figure 11). The tumour size continued to decrease until day 18 after which the tumour volume began to increase (perhaps due to the entire drug being eluted from the beads). The body weight of the doxorubicin treated mice increased from 20g to 25g; this can be used as an indication no toxicity (Figure 12). A decrease in body weight occurred from day 21 to 25 (an indication of toxicity); this is likely to be due to tumour growth since no weight loss occurred 11 days after treatment with doxorubicin. The doxorubicin bead treated mice were euthanized after the 25 day time point because the tumour was greater than 10% of the total weight of the

mouse.

**Comparative Example 8: In vivo Direct (Repeat) Injection of Irinotecan or Topotecan Loaded Beads into Human Pancreatic Carcinoma Xenograft on Nude Mice.**

*Pre-treatment:* PSN-1 tumour pieces (human pancreatic carcinoma) were prepared from an in vivo passage for subcutaneous transplantation. Mice were randomised to the scheduled treatment groups.

*Anaesthesia:* None

*Clinical Procedure:* After six days the tumours became palpable (2-3 mm) and the DEB / alginate mixture was injected close to the subcutaneous growing tumour. Prior to injection, all of the lyophilised sterilised Drug Eluting Beads (DEB) were hydrated on the day of administration by the addition of 3 ml alginate solution.

The volumes injected and drug loading are shown in Table 2. Repeat injections were made when the mean tumour size was ≥20% larger than the previous time point. Repeat injections were performed for group B at 22 and 36 days; group C at 19 days; group D at 19 and 27 days; and group E at 19, 27 and 36 days.

The body weight of each mouse and the size of the tumour was determined and recorded twice a week.

Table 2: Group information for mouse xenograft study

| Group | Drug loaded | Amount of drug loaded per mL of beads | Total volume (alginate and DEB) injected (μL) | Dose (mg) |
|---|---|---|---|---|
| A | Unloaded | 0 | 100 | 0 |
| B | Irinotecan | 100 | 100 | 3.3 |
| C | | 100 | 200 | 6.6 |
| D | Topotecan | 12 | 100 | 0.4 |
| E | | 6 | 100 | 0.2 |

*Results:* The growth of PSN1 tumours was inhibited with topotecan and irinotecan DEBs administered subcutaneously with alginate in nude mice (Figure 13). The control (- -) in both cases were unloaded beads in alginate solution. Repeat injections were made for 3.3 mg irinotecan (22 and 36 days); 6.6 mg irinotecan (19 days); 0.4 mg topotecan (19 and 27 days) and 0.2 mg topotecan (19, 27 and 36 day). (n=3 mean +/- max / min).

The topotecan concentrations (0.4 and 0.2 mg) showed efficacy in reducing the tumour volume. The irinotecan treatments were equal or better than the topotecan treatments tested in reducing tumour size.

The control mice showed severe body weight loss when tumours reached a larger tumour volume, which was attributed to tumour induced cachexia. Based on this measure of toxicity all groups showed no significant weight loss, indicating that the treatments were well tolerated (Figure 14).

A much lower dose of topotecan was required to elicit a cytotoxic effect compared to irinotecan. From all of the samples tested here, the higher irinotecan dose group showed the greatest potential surviving for 57 days post the tumour implant. Two mice from this group demonstrated significant tumour shrinkage, with one demonstrating >99% shrinkage after 47 days and no signs of regrowth after 57 days. The variability in the groups may be due to inconsistencies in the injection sites. In the higher irinotecan dose treatment, an increase in tumour size can be observed after 19 days. This could either be due to drug resistance or insufficient drug. The reduction in tumour size after the second injection demonstrates that the growth is due to irinotecan fully eluting from the beads and not drug resistance (Figure 13).

**Comparative Example 9: Endoscopic Ultrasound-Guided Injection of Irinotecan Loaded**

**Beads in Alginate in a Porcine Model**

*Endoscopic ultrasonography (EUS):* EUS is a medical procedure in which endoscopically directed ultrasound is used to obtain images of the internal organs or tumours in the chest and abdomen.

*Procedure:* A EUS scope (13 mm in diameter) was inserted into the oesophagus of anesthetized pigs via the mouth and passed to the proximal stomach of all pigs. Irinotecan DEBs / alginate mixture were prepared for direct injection into the pancreas. Increasing volumes of irinotecan beads were mixed and suspended in the alginate solution to allow for different doses of irinotecan to be injected. The irinotecan concentrations ranged from 50 to 300 mg (n =1-2).

The control pigs were injected with 2 mL unloaded DEBs suspending in 2 ml, alginate solution. The pancreas was entered with a 19 gauge needle under EUS guidance and catheter (pre¬wetted with saline). A 4 mL volume of alginate solution containing the required volume of irinotecan DEBs was injected into the pancreas. A CT scan was taken post procedure. The all pigs were monitored for 7 days after the procedure. The animals underwent necropsy at end of the 7th day. The pancreatic tissue was analysed by a histopathologic examination and gross pathology. At necropsy, the average diameter of the injection site was 25.4 $\pm$ 4.2 (20-30) mm.

After microsphere injection, an intrapancreatic hyperechoic focus was clearly visible by EUS. The site of injection was apparent on CT scanning as a hypodense area in the tail of the pancreas (see Figure 15).

All pigs showed no clinical signs of pancreatitis or intra-abdominal infection by clinical, radiologic, or gross pathology up to a dose of 300 mg irinotecan.

Histopathologic examination confirmed the presence of a foreign body giant cell reaction and granulation tissue around the beads. There was mild inflammation surrounding these areas both control and drug eluting beads group. The degree of inflammation was similar whether the beads contained irinotecan or not (Figure 16).

**Reference Documents**

1. http://pancan.org/About/pancreaticCancerStats.html
2. Ahmedin Jemal, DVM,PhD, Rebecca Siegel, MPH, Elizabeth Ward, PhD, Taylor Murray, Jiaquan Xu and Michael J. Thun, MD,MS. Cancer Statistics, 2007. CA Cancer J Clin 2007; 57:43-66
3. Stephens J, Kuhn J, O'Brien J, et al. Surgical morbidity, mortality, and long-term survival in patients with peripancreatic cancer following pancreaticoduodenectomy. Am J Surg. 1997;174: 600-604.
4. Andersen JR, Friis-Möller A, Hancke S, Röder O, Steen J, Baden H. A controlled trial of combination chemotherapy with 5-FU and BCNU in pancreatic cancer. Scand J Gastroenterol 1981;16:973-5.
5. Frey C, Twomey P, Keehn R, Elliott D, Higgins G. Randomized study of 5-FU and CCNU in pancreatic cancer: report of the Veterans Administration Surgical Adjuvant Cancer Chemotherapy Study Group. Cancer 1981;47:27-31.
6. Mallinson CN, Rake MO, Cocking JB, Fox CA, Cwynarski MT, Diffey BL, et al. Chemotherapy in pancreatic cancer: results of a controlled, prospective, randomised, multicentre trial. BMJ 1980;281:1589-91.
7. Moertel CG, Frytak S, Hahn RG, O'Connell MJ, Reitemeier RJ, Rubin J, et al. Therapy of locally unresectable pancreatic carcinoma: a randomized comparison of high dose (6000 rads) radiation alone, moderate dose radiation (4000 rads 1 5-fluorouracil), and high dose radiation 1 5-fluorouracil: the Gastrointestinal Tumour Study Group. Cancer 1981;48:1705-10.
8. Gastrointestinal Tumour Study Group. Radiation therapy combined with Adriamycin or 5-fluorouracil for the treatment of locally unresectable pancreatic carcinoma. Cancer 1985;56:2563-8.
9. Klaassen DJ, Macintyre JM, Catton GE, Engstrom PF, Moertel CG. Treatment of locally unresectable cancer of the stomach and pancreas: a randomized comparison of 5-fluorouracil alone with radiation plus concurrent and maintenance 5-fluorouracil-an Eastern Cooperative Oncology Group study. J Clin Oncol 1985;3:373-8.
10. Gastrointestinal Tumour Study Group. Treatment of locally unresectable carcinoma of the pancreas: comparison of combined-modality therapy chemotherapy plus radiotherapy to chemotherapy alone. J Natl Cancer Inst 1988;80:751-5.
11. Burris HA III, Moore MJ, Andersen J, Green MR, Rothenberg ML, Modiano MR, et al. Improvements in survival and clinical benefit with gemcitabine as first-line therapy for patients with advanced pancreas cancer: cancer: a randomized trial. J Clin Oncol 1997;15:2403-13.
12. Lorenz M, Heinrich S, Staib-Sebler E, Kohne Cho, Vllils J, Nordlinger B, Encke A. Regional chemotherapy in the treatment of advanced pancreatic cancer - is it relevant? Eur J Cancer. 2000 May;36(8):957-65.
13. Nirag C. Jhala, Darshana N. Jhal, David C. Chhieng, Mohamad A. Eloubeidi, Isam A. Eltoum, Isam A. Eltoum, (Am J Clin Pathol 120(3):351-367, 2003).
14. Anthony J. Dimarino, Jr., Anthony J. DiMarino, Stanley B. Benjamin Gastrointestinal Disease: An Endoscopic Approach, Published 2002: 1078 ISBN 1556425112
15. Kenneth J. Chang, M.D., Phuong T. Nguyen, M.D., James A. Thompson, M.D., Thomas T. Kurosaki, M.S., Linda R. Casey, M.D., Edwin C. Leung, M.A., Gale A. Granger, Ph.D. Phase I clinical trial of allogeneic mixed lymphocyte culture (cytoimplant) delivered by endoscopic ultrasound-guided fine-needle injection in patients with advanced pancreatic carcinoma. Cancer, Volume 88, Issue 6 , Pages 1325 - 1335.
16. Oh HC, Seo DW, Lee TY, Kim JY, Lee SS, Lee SK, Kim MH. New treatment for cystic tumours of the pancreas: EUS-guided ethanol lavage with paclitaxel injection. Gastrointest Endosc. 2008 Apr;67(4):636-42.
17. Mattsson J, Naredi P, Hafström L, Peterson HI. Intratumoural distribution of microspheres. Anticancer Res. 1986 Jul-Aug;6(4):563-6.

18. Gupta PK, Hung, CT, Lam FC, Application of regression analysis in the evaluation of tumour response following the administration of adriamycin as a solution or via albumin microspheres to the rat. J Pharm Sci. 1900; 79(7): 634-7.
19. Almond BA, Hadba AR, Freeman ST, Cuevas BJ, York AM, Detrisac CJ, Goldberg EP. Efficacy of mitoxantrone-loaded albumin microspheres for intratumoural chemotherapy of breast cancer. J Control Release. 2003 Aug 28;91 (1-2):147-55.
20. Emerich DF, Snodgrass P, Lafreniere D, Dean RL, Salzberg H, Marsh J, Perdomo B, Arastu M, Winn SR, Bartus RT Sustained release chemotherapeutic microspheres provide superior efficacy over systemic therapy and local bolus infusions. Pharm Res. 2002 Jul;19(7):1 052-60.
21. Liu Z, Ballinger JR, Rauth AM, Bendayan R, Wu XY. Delivery of an anticancer drug and a chemosensitizer to murine breast sarcoma by intratumoural injection of sulfopropyl dextran microspheres. J Pharm Pharmacol. 2003 Aug;55(8):1063-73.
22. Sun S et al. EUS-guided interstitial chemotherapy in the pancreas Endoscopy 2007; 39:530-534

## Claims

1. A composition comprising microspheres which comprise a water insoluble, water-swellable polymer and associated with the polymer, in releasable form, a first and second chemotherapeutic agent, for use in the treatment of a pancreatic tumour or cyst, the first chemotherapeutic agent having the general formula I

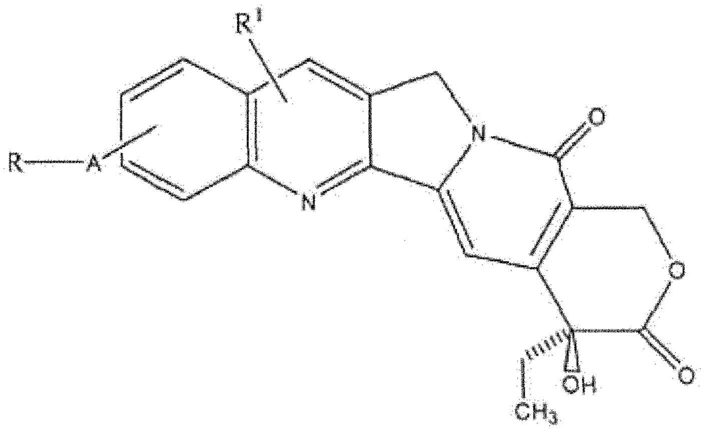

I

in which $R^1$ is selected from H, halogen, hydroxyl and lower ($C_{1-6}$) alkyl, optionally substituted by a hydroxyl, amine, alkoxy, halogen, acyl and acyloxy groups;
A is C(O)O or $CH_2$; and
R is $NR^2R^3$ where $R^2$ and $R^2$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form an optionally substituted heterocyclic ring which may be interrupted by -O-, -S- or $>NR_4$ in which $R^4$ is a hydrogen atom, a substituted or unsubstituted alkyl group or a substituted or unsubstituted phenyl group; and wherein the grouping -A-R is bonded to a carbon atom located in any of the positions in the A ring of the camptothecin compound and $R^1$ is substituted in the A or B ring, including salts thereof;
and the second chemotherapeutic agent is selected from rapamycin and its analogues, which analogues are selected from sirolimus, temsirolimus, everolimus, biolimus, ABT-578 and AP23573 and esters and salts thereof; wherein the microspheres, when equilibrated in water at 37°C, comprise at least 40 wt% water based on weight of polymer plus water, wherein the polymer is anionically charged at pH7 and the first chemotherapeutic agent is cationically charged and electrostatically associated with the polymer, and wherein separate particles are, each loaded with one of the drugs or each particle is jointly loaded with both the drugs;
and wherein, in the treatment, the composition is injected into the pancreatic tumour or cyst or around its perimeter, preferably under the guidance of endoscopic ultrasound (EUS).

2. Composition for use according to claim 1 wherein the first chemotherapeutic agent is irinotecan.

3. A composition for use according to claim 1, wherein the rapamycin analogues, esters and salts have water solubility of less than 0.1 g/l at room temperature.

4. A composition for use according to any preceding claim, in which the polymer is alginate, polyvinyl alcohol or cross linked polyvinyl alcohol.

5. A composition for use according to claim 4, in which the polymer is formed from polyvinyl alcohol (PVA) macromer, having more than one ethylenically unsaturated pendant group per molecule, by radical polymerisation of the ethylenic groups.

6. A composition for use according to claim 5, in which the macromer is copolymerised with ethylenically unsaturated monomers for instance including a nonionic and/or ionic monomer including anionic monomer.

7. A composition for use according to claim 6, in which the ethylenically unsaturated monomers include ionic monomer having the general formula III

$$Y^1BQ^1 \qquad III$$

in which $Y^1$ is selected from

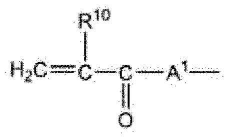

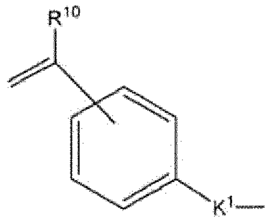

$CH_2=C(R^{10})-CH_2-O-$, $CH_2=C(R^{10})-CH_2$ $OC(O)-$, $CH_2=C(R^{10})OC(O)-$, $CH_2=C(R^{10})-O-$, $CH_2=C(R^{10})CH_2OC(O)N(R^{11})-$, $R^{12}OOCCR^{10}=CR^{10}C(O)-O-$, $R^{10}CH=CHC(O)O-$, $R^{10}CH=C(COOR^{12})CH_2C(O)-O-$,

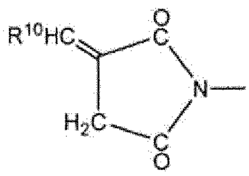

and

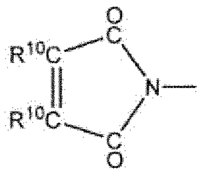

wherein:

$R^{10}$ is hydrogen or a $C_1$-$C_4$ alkyl group;
R" is hydrogen or a $C_1$-$C_4$ alkyl group;
$R^{12}$ is hydrogen or a Alkyl group or $BQ^1$ where B and $Q^1$ are as defined below;
$A^1$ is -O- or $-NR^{11}-$;
$K^1$ is a group $-(CH_2)_rOC(O)-$, $-(CH_2)_rC(O)O-$, - $(CH_2)_rOC(O)O-$,$-(CH_2)_rNR^{13}$, $-(CH_2)_rNR^{13}C(O)-$, $-(CH_2)_rC(O)NR^{13}-$, $-(CH_2)_rNR^{13}C(O)O-$,$-(CH_2)_rOC(O)NR^{13}-$, $-(CH_2)_rNR^{13}C(O)NR^{13}-$ (in which the groups $R^{13}$ are the same or different), $-(CH_2)_rO-$, $-(CH_2)_rSO3$ -, or, optionally in combination with B, a valence bond and r is from 1 to 12 and $R^{13}$ is hydrogen or a $C_1$-$C_4$ alkyl group; B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if $Q^1$ or $Y^1$ contains a terminal carbon atom bonded to B a valence bond; and $Q^1$ is an anionic group.

8. A composition for use according to claim 1 the composition comprising microspheres which comprise a water insoluble, water-swellable polymer selected from cross linked polyvinyl alcohol and a copolymer of vinyl alcohol and an anionic acrylic monomer, and associated with the polymer, in releasable form, a first and second chemotherapeutic agent,
wherein the first chemotherapeutic agent, is irinotecan, which is cationically charged and electrostatically associated with the polymer and the second chemotherapeutic agent is rapamycin,
and wherein separate particles are each loaded with one of the drugs or each particle is jointly loaded with both the

drugs.

9. A composition for use according to any preceding claim which comprises a viscosity modifier, preferably alginate.

10. A composition for use according to any preceding claim which is a pharmaceutical composition and further comprises one or more pharmaceutically acceptable excipients.

11. A composition for use according to claim 1, wherein separate particles are each loaded with one of the drugs.

**Patentansprüche**

1. Zusammensetzung, umfassend Mikrokügelchen, die ein wasserunlösliches, wasserquellbares Polymer umfassen und mit dem Polymer verbunden, in freisetzbarer Form, ein erstes und zweites chemotherapeutisches Mittel zur Verwendung bei der Behandlung eines/einer Bauchspeicheldrüsentumors oder -zyste, wobei das erste chemotherapeutische Mittel die allgemeine Formel I aufweist

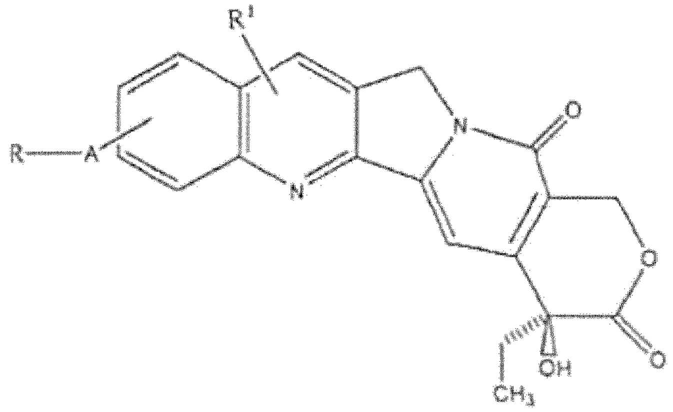

I

wobei $R^1$ aus H, Halogen, Hydroxl und Nieder-($C_{1-6}$)Alkyl ausgewählt ist, gegebenenfalls mit Hydroxyl-, Amin-, Alkoxy-, Halogen-, Acyl- und Acyloxygruppen substituiert ist;
A C(O)O oder $CH_2$ ist; und
R $NR^2R^3$ ist, wobei $R^2$ und $R^3$ gleich oder verschieden sind und jeweils für ein Wasserstoffatom, eine substituierte oder nichtsubstituierte $C_{1-4}$-Alkylgruppe oder eine substituierte oder nichtsubstituierte carbocyclische oder heterocyclische Gruppe stehen, oder $R^2$ und $R^3$ zusammen mit dem Stickstoffatom, an das sie gebunden sind, einen optional substituierten heterocyclischen Ring bilden, der durch -O-, -S- oder $>NR_4$ unterbrochen sein kann, wobei $R^4$ ein Wasserstoffatom, eine substituierte oder nichtsubstituierte $C_{1-4}$-Alkylgruppe oder eine substituierte oder nichtsubstituierte Phenylgruppe ist; und wobei die Gruppierung -A-R an ein Kohlenstoffatom gebunden ist, das sich in einer der Positionen im A-Ring der Camptothecinverbindung befindet and $R^1$ in dem A- oder B-Ring substituiert ist, einschließlich Salze davon;
und das zweite chemotherapeutische Mittel aus Rapamycin und dessen Analoga ausgewählt ist, wobei die Analoga aus Sirolimus, Temsirolimus, Everolimus, Biolimus, ABT-578 und AP23573 und Estern und Salzen davon ausgewählt sind;
wobei die Mikrokügelchen, wenn in Wasser bei 37 °C äquilibriert, zumindest 40 Gew.-% Wasser, basierend auf dem Gewicht des Polymers plus Wasser umfassen, wobei das Polymer bei pH-Wert 7 anionisch geladen ist und das erste chemotherapeutische Mittel kationisch geladen ist und elektrostatisch mit dem Polymer assoziiert ist, und wobei separate Partikel jeweils mit einem der Arzneimittel beladen sind oder jedes Partikel mit beiden der Arzneimittel gemeinsam beladen ist;
und wobei die Zusammensetzung bei der Behandlung in den/die Bauchspeicheldrüsentumor oder - zyste oder um seinen/ihren Umfang injiziert wird, vorzugsweise unter der Führung eines endoskopischen Ultraschalls (EUS).

2. Zusammensetzung zur Verwendung nach Anspruch 1, wobei das erste chemotherapeutische Mittel Irinotecan ist.

**3.** Zusammensetzung zur Verwendung nach Anspruch 1, wobei die Rapamycin-Analoga, -Ester und -Salze eine Wasserlöslichkeit von weniger als 0,1 g/l bei Raumtemperatur aufweisen.

**4.** Zusammensetzung zur Verwendung nach einem der vorhergehenden Ansprüche, wobei das Polymer Alginat, Polyvinylalkohol oder vernetzter Polyvinylalkohol ist.

**5.** Zusammensetzung zur Verwendung nach Anspruch 4, wobei das Polymer aus Polyvinylalkohol(PVA)-Makromer, aufweisend mehr als eine ethylenisch ungesättigte Seitengruppe pro Molekül, durch radikalische Polymerisation der ethylenischen Gruppen gebildet ist.

**6.** Zusammensetzung zur Verwendung nach Anspruch 5, wobei das Makromer mit ethylenisch ungesättigten Monomeren copolymerisiert ist, die zum Beispiel ein nichtionisches und/oder ionisches Monomer einschließen, einschließlich eines anionischen Monomers.

**7.** Zusammensetzung zur Verwendung nach Anspruch 6, wobei die ethylenisch ungesättigten Monomere ein ionisches Monomer einschließen, das die allgemeine Formel III aufweist

$Y^1BQ^1$ III

wobei $Y^1$ ausgewählt ist aus

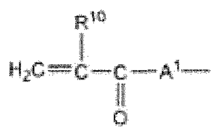

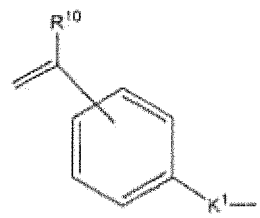

$CH_2=C(R^{10})-CH_2-O-$, $CH_2=C(R^{10})-CH_2$ $OC(O)-$, $CH_2=C(R^{10})OC(O)-$, $CH_2=C(R^{10})-O-$, $CH_2=C(R^{10})CH_2OC(O)N(R^{11})-$, $R^{12}OOCCR^{10}=CR^{10}C(O)-O-$, $R^{10}CH-CHC(O)O-$, $R^{10}CH=C(COOR^{12})CH_2-C(O)-O-$,

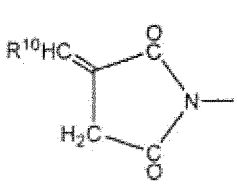

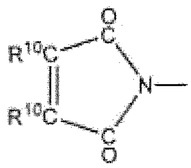

und

wobei:

$R^{10}$ Wasserstoff oder eine $C_1$-$C_4$-Alkylgruppe ist;
$R^{11}$ Wasserstoff oder eine $C_1$-$C_4$-Alkylgruppe ist;
$R^{12}$ Wasserstoff oder eine $C_{1-4}$-Alkylgruppe oder $BQ^1$ ist, wobei B und $Q^1$ wie folgt definiert sind;
$A^1$ -O- oder $NR^{11}$- ist;
$K^1$ eine Gruppe $-(CH_2)_rOC(O)-$, $-(CH_2)_rC(O)O-$, $-(CH_2)_rOC(O)O-$, $-(CH_2)_rNR^{13}$, $-(CH_2)_rNR^{13}C(O)-$, $-(CH_2)_rC(O)NR^{13}-$, $-(CH_2)_rNR^{13}C(O)O-$, $-(CH_2)_rOC(O)NR^{13}-$, $-(CH_2)_rNR^{13}C(O)NR^{13}-$ ist, (bei der die $R^{13}$-Gruppen gleich oder verschieden sind), $-(CH_2)_rO-$,$-(CH_2)$,SO3-, oder, optional in Kombination mit B, eine Valenzbindung ist und r von 1 bis 12 ist und $R^{13}$ ein Wasserstoff oder eine $C_1$-$C_4$-Alkylgl-uppe ist; B eine gerade oder verzweigte Alkandiyl-, Oxaalkylen-, Alkandiyloxaalkandiyl- oder eine Alkandiyloligo(oxaalkandiyl)-Kette ist, die optional ein oder mehrere Fluoratom(e) bis zu und einschließlich perfluorierten Ketten enthält oder eine Valenzbindung, wenn $Q^1$ oder $Y^1$ ein endständiges Kohienstoffatom enthält, das an B gebunden ist;
und $Q^1$ eine anionische Gruppe ist.

**8.** Zusammensetzung zur Verwendung nach Anspruch 1, wobei die Zuaammensetzung Mikrokiigelehen umfasst, die ein wasserlöslicltes, wasserquellbares Polymer umfassen, das aus einem vernetzten Polyvinylalkohol und einem Copolymer von Vinylalkohol und einem anionischen Acrylmavorner ausgewählt ist und mit dem Polymer assoziiert, in freisetzbarer Form, ein erstes und zweites chemotherapeutisches Mittel,
wobei das erste chemotherapeutische Mittel Irinotecan ist, das kationisch geladen ist und elektrostatisch mit dem Polymer assoziiert ist und das zweite chemotherapeutische Mittel Rapamycin ist,
und wobei separate Partikel jeweils mit einem der Arzneimittel beladen sind oder jedes Partikel mit beiden der Arzneimittel gemeinsam beladen ist.

**9.** Zusammensetzung zur Verwendung nach einem der vorhergehenden Ansprüche, die einen Viskositätsmodifizierer, vorzugsweise Alginat, umfasst.

**10.** Zusammensetzung zur Verwendung nach einem der vorhergehenden Ansprüche, die eine pharmazeutische Zusammensetzung ist und ferner einen oder mehrere pharmazeutisch unbedenkliche Exzipienten umfasst.

**11.** Zusammensetzung zur Verwendung nach Anspruch 1, wobei separate Partikel jeweils mit einem der Arzneimittel beladen sind.

## Revendications

**1.** Composition comprenant des microsphères qui comprennent un polymère insoluble dans l'eau, capable de gonfler dans l'eau et, associés au polymère, sous une forme libérable, un premier et un second agent de chimiothérapie, à utiliser dans le traitement d'une tumeur ou d'un kyste du pancréas, le premier agent de chimiothérapie ayant la formule générale I

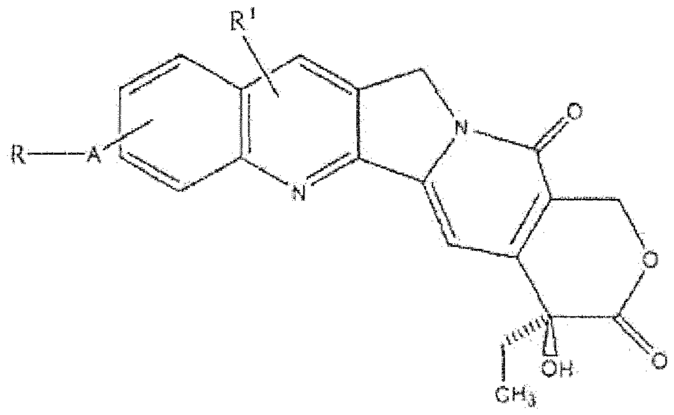

I

dans laquelle $R^1$ est sélectionné parmi un groupe H, halogène, hydroxyle et alkyle intérieur ($C_{1-6}$), facultativement substitué par un groupe hydroxyle, amine, alcoxy, halogène, acyle et acyloxy ;
A est C(O)O ou $CH_2$ ; et
R est $NR^2R^3$ dans lequel $R^2$ et $R^3$ sont identiques ou différents et chacun représente un atome d'hydrogène, un groupe alkyle $C_{1-4}$ substitué ou non substitué ou un groupe carbocyclique ou hétérocyclique substitué ou non substitué, ou $R^2$ et $R^3$, ensemble avec l'atome d'azote auquel ils sont fixés, forment un anneau hétérocyclique facultativement substitué qui peut être interrompu par -O-, -S- ou >$NR_4$ dans lequel $R^4$ est un atome d'hydrogène, un groupe alkyle $C_{1-4}$ substitué ou non substitué ou un groupe phényle substitué ou non substitué ; et dans laquelle le groupe -A-R est relié à un atome de carbone situé dans l'une quelconque des positions dans l'anneau A du composé camptothécine et $R^1$ est substitué dans l'anneau A ou B, incluant ses sels ;
et le second agent de chimiothérapie est sélectionné parmi la rapamycine et ses analogues, lesquels analogues sont sélectionnés parmi le sirolimus, le temsirolimus, l'évérolimus, le biolimus, ABT-578 et AP23573 et leurs esters et sels ;
dans laquelle les microsphères, lorsqu'elles sont équilibrées dans l'eau à 37 °C, comprennent au moins 40 % d'eau en poids sur la base du poids du polymère plus l'eau, dans laquelle le polymère est chargé anioniquement à un pH de 7 et le premier agent de chimiothérapie est chargé cationiquement et associé électrostatiquement au polymère, et dans laquelle des particules séparées sont chacune chargées avec l'un des médicaments ou

chaque particule est chargée conjointement avec les deux médicaments ;
et dans laquelle, dans le traitement, la composition est injectée dans la tumeur ou le kyste du pancréas ou autour de son périmètre, de préférence sous guidage par échographie endoscopique (EES).

**2.** Composition à utiliser selon la revendication 1, dans laquelle le premier agent de chimiothérapie est l'irinotécan.

**3.** Composition à utiliser selon la revendication 1, dans laquelle les analogues, esters et sels de la rapamycine présentent une solubilité dans l'eau inférieure à 0,1 g/l à température ambiante.

**4.** Composition à utiliser selon l'une quelconque des revendications précédentes, dans laquelle le polymère est de l'alginate, de l'alcool polyvinylique ou de l'alcool polyvinylique réticulé.

**5.** Composition à utiliser selon la revendication 4, dans laquelle le polymère est formé à partir d'un macromère d'alcool polyvinylique (PVA), ayant plus d'un groupe latéral éthyléniquement insaturé par molécule, par polymérisation radicalaire des groupes éthyléniques.

**6.** Composition à utiliser selon la revendication 5, dans laquelle le macromère est copolymérisé avec des monomères éthyléniquement insaturés incluant par exemple un monomère non ionique et/ou ionique incluant un monomère anionique.

**7.** Composition à utiliser selon la revendication 6, dans laquelle les monomères éthyléniquement insaturés inclurent un monomère ionique ayant la formule générale III

$$Y^1BQ^1 \qquad III$$

dans laquelle $Y^1$ est sélectionné parmi

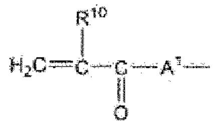

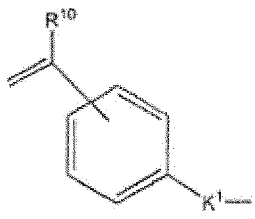

$CH=C(R^{10})-CR_2-O-$, $CH_2=C(R^{10})-CH_2$ $OC(O)-$, $CH_2=C(R^{10})OC(O)-$, $CH_2=C(R^{10})-O-$, $CH_2=C(R^{10})CH_2OC(O)N(R^{11})-$, $R^{12}OOCCR^{10}=CR^{10}C(O)-O-$, $R^{10}CH=CHC(O)O-$, $R^{10}CH=C(COOR^{12})CH_2-C(O)-O-$,

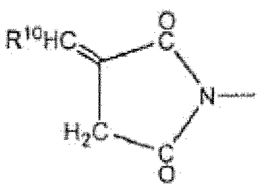

et

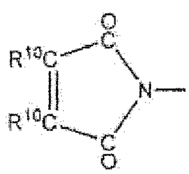

dans lesquelles :

$R^{10}$ est un hydrogène ou un groupe alkyle C1-$C_4$ ;
$R^{11}$ est un hydrogène ou un groupe alkyle $C_1$-$C_4$ ;
$R^{12}$ est un hydrogène ou un groupe alkyle $C_{1-4}$ ou $BQ^1$ dans laquelle B et $Q^1$ sont comme définis ci-dessous ;
$A^1$ est -O- ou $-NR^{11}-$;
$K^1$ est un groupe - $(CH_2)rOC(O)-$, $-(CH_2)_rC(O)O-$,$-(CH_2)_rOC(O)O-$, $-(CH_2)_rNR^{13}$, $-(CH_2)_rNR^{13}C(O)-$, - $(CH_2)_rC(O)NR^{13}-$,$-(CH_2)_rNR^{13}C(O)O-$, - $(CH_2)_rOC(O)NR^{13}-$, - $(CH_2)_rNR^{13}C(O)NR^{13}-$ (dans lesquels les groupes $R^{13}$ sont identiques ou différents), $-(CH_2)_rO-$, $-(CH_2)_rSO3-$ ou, facultativement en association avec B, une liaison de valence, et r est compris entre 1 et 12 et $R^{13}$ est un hydrogène ou un groupe alkyle $C_1$-$C_4$ B est une chaîne alcanediyle, oxaalkylène, alcanediyle-oxaalkylène ou alcanediyle-oligo(oxaalkynédiyle) droite ou ramifiée contenant facultativement un ou plusieurs atomes de fluor jusqu'à et y compris des chaînes perfluorées ou, si $Q^1$ ou $Y^1$ contient un atome de carbone terminal lié à B par une liaison de valence ; et $Q^1$ est un groupe anionique.

**8.** Composition à utiliser selon la revendication 1, la composition comprenant des microsphères qui comprennent un polymère insoluble dans l'eau, capable de gonfler dans l'eau, sélectionné parmi un alcool polyvinylique réticulé et un copolymère d'alcool vinylique et un monomère d'acrylique anionique, et, associés au polymère, sous forme libérable, un premier et un second agents de chimiothérapie,
dans laquelle le premier agent de chimiothérapie est l'irinotécan, qui est chargé cationiquement et associé électrostatiquerrient avec le polymère, et le second agent de chimiothérapie est la rapamycine,
et dans laquelle des particules séparées sont chacune chargées avec l'un des médicaments ou chaque particule est chargée conjointement avec les deux médicaments.

**9.** Composition à utiliser selon l'une quelconque des revendications précédentes, qui comprend un modificateur de viscosité, de préférence de l'alginate.

**10.** Composition à utiliser selon l'une quelconque des revendications précédentes qui est une composition pharmaceutique et comprend en outre un ou plusieurs excipients pharmaceutiquement acceptables.

**11.** Composition à utiliser selon la revendication 1, dans laquelle des particules séparées sont chacune chargées avec un des médicaments.

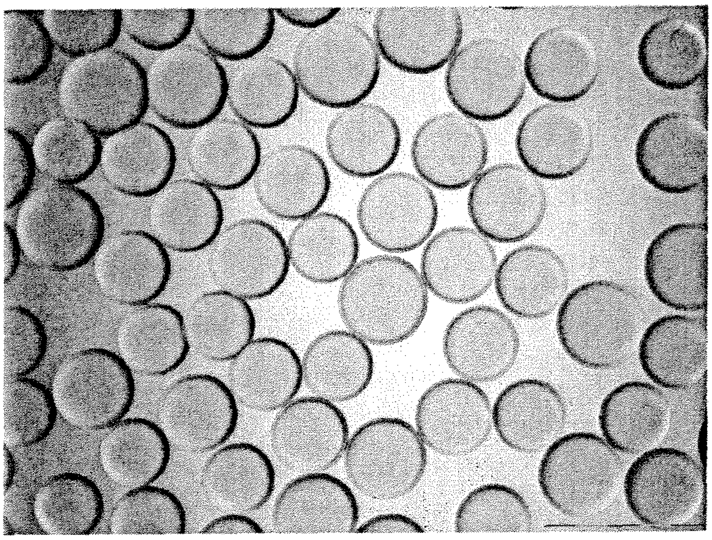

Figure 1

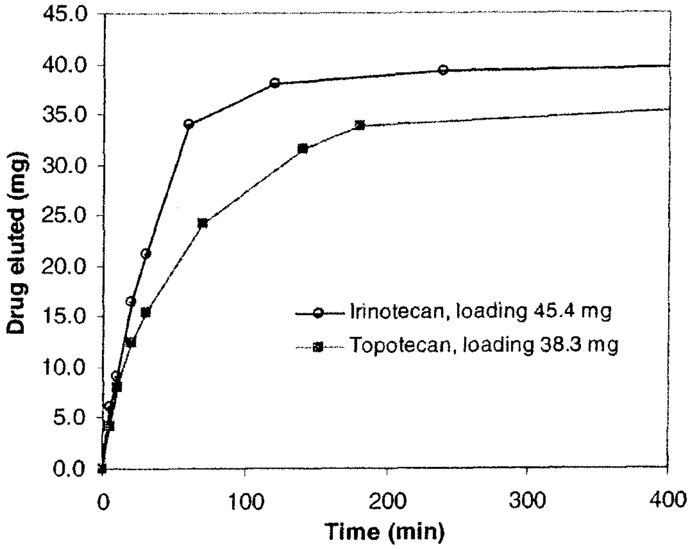


Figure 2

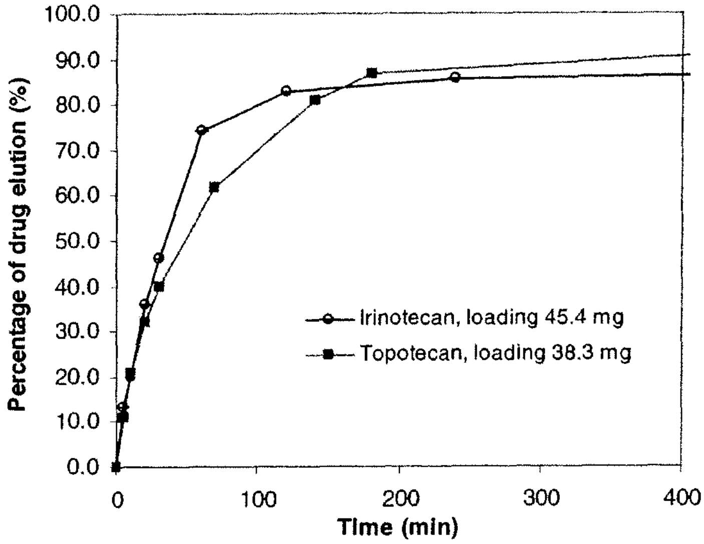


**Figure 3**

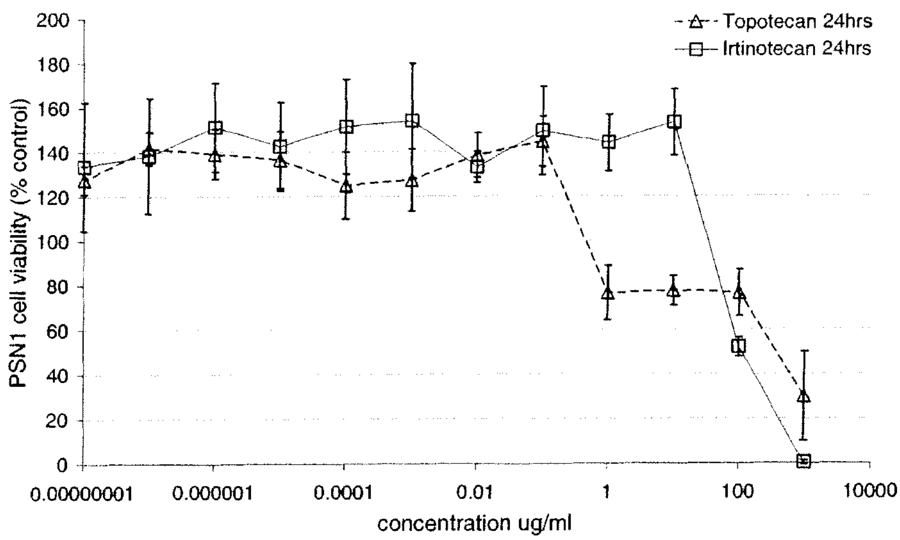


**Figure 4**

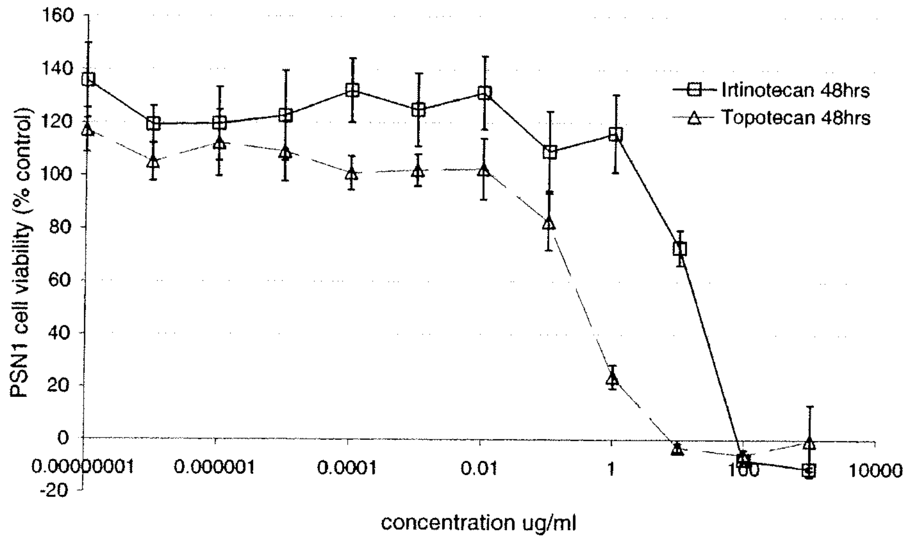


**Figure 5**

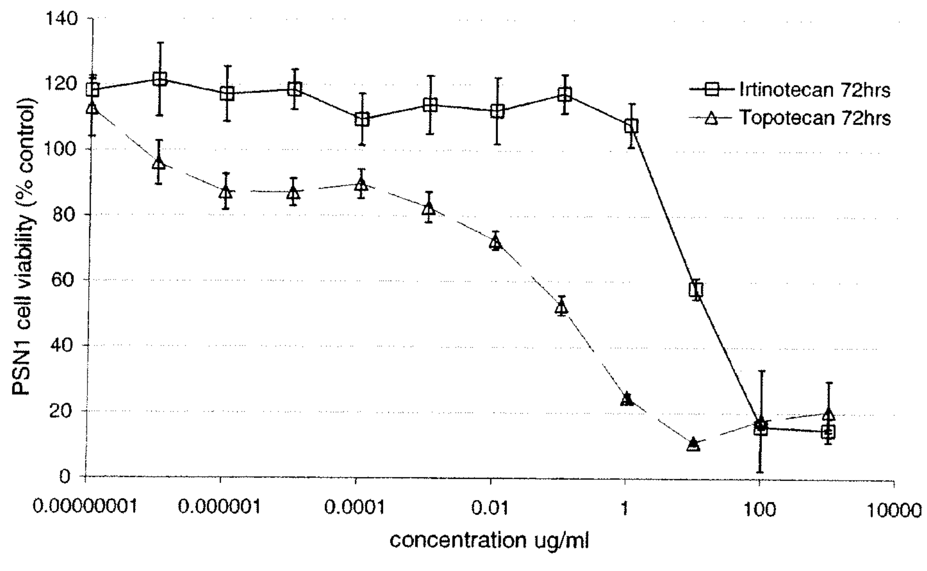


**Figure 6**

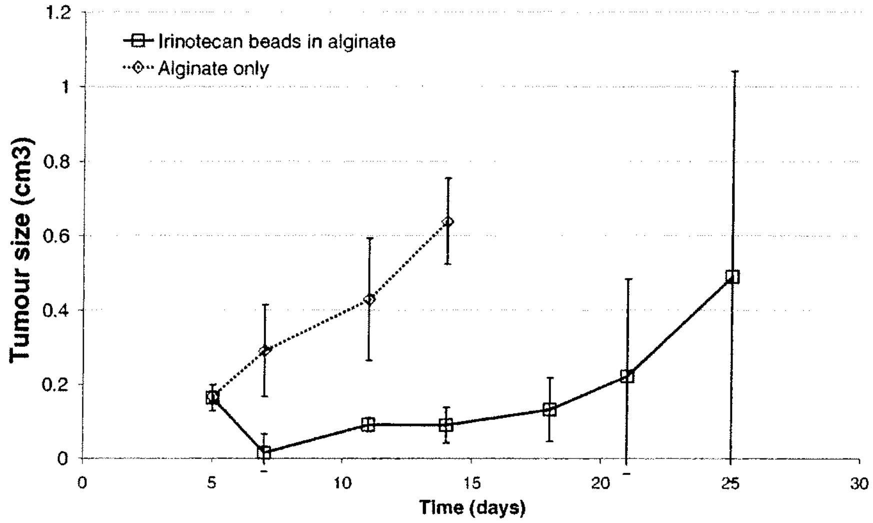


Figure 7

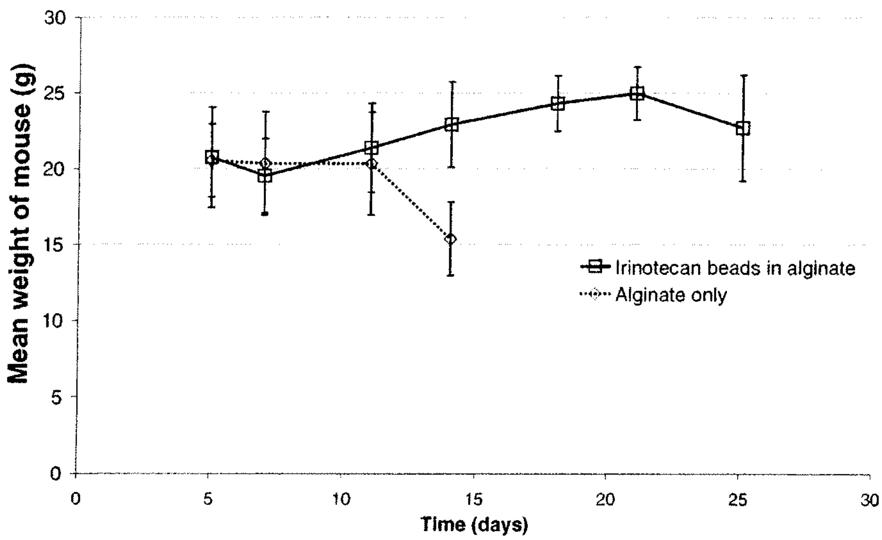


Figure 8

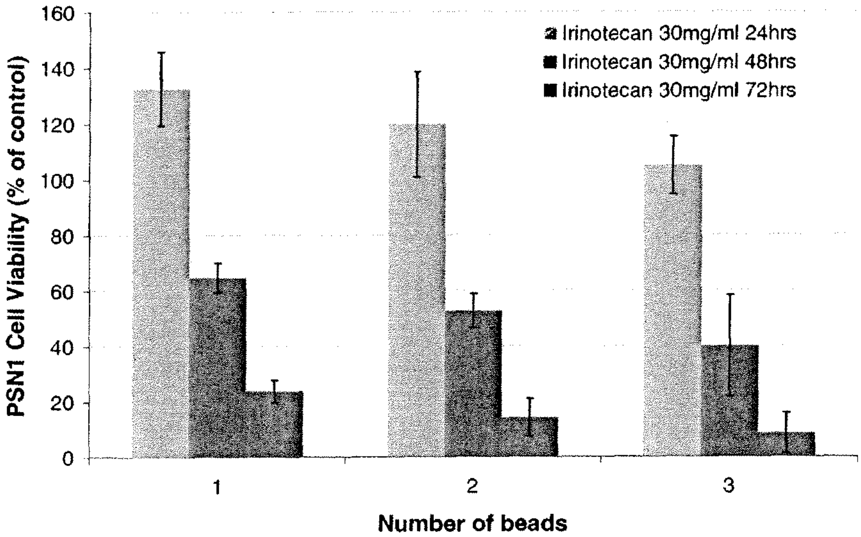


Figure 9

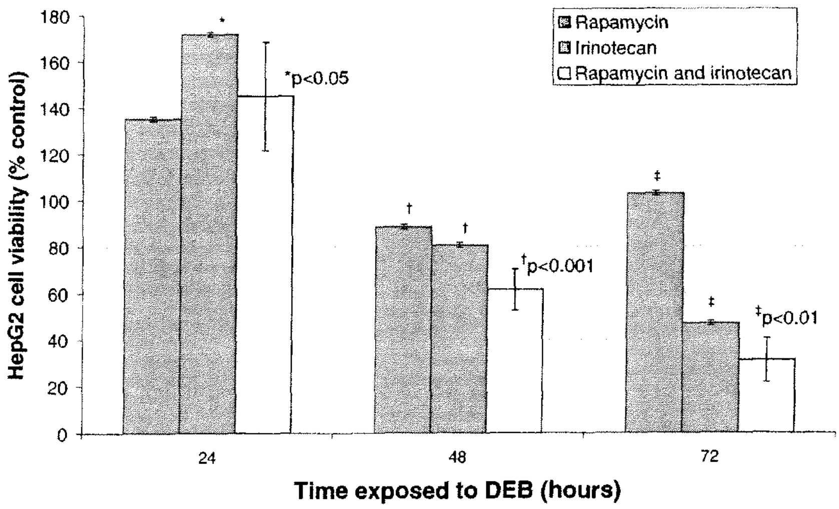


Figure 10

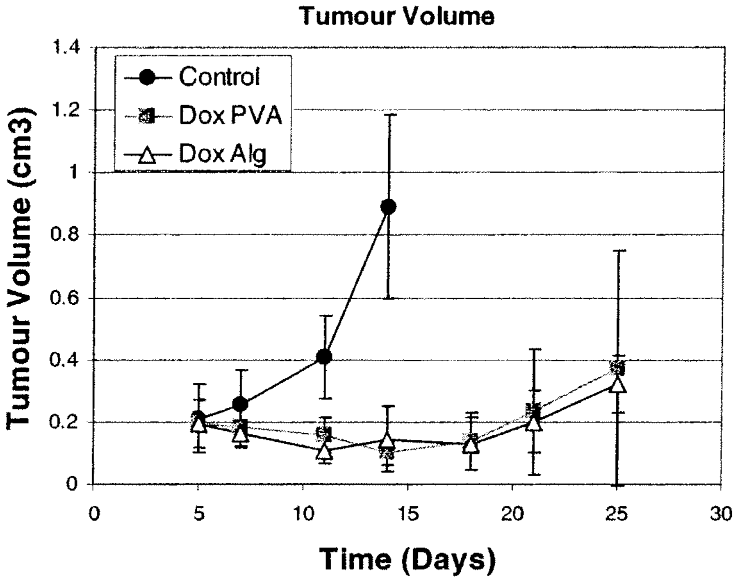


Figure 11

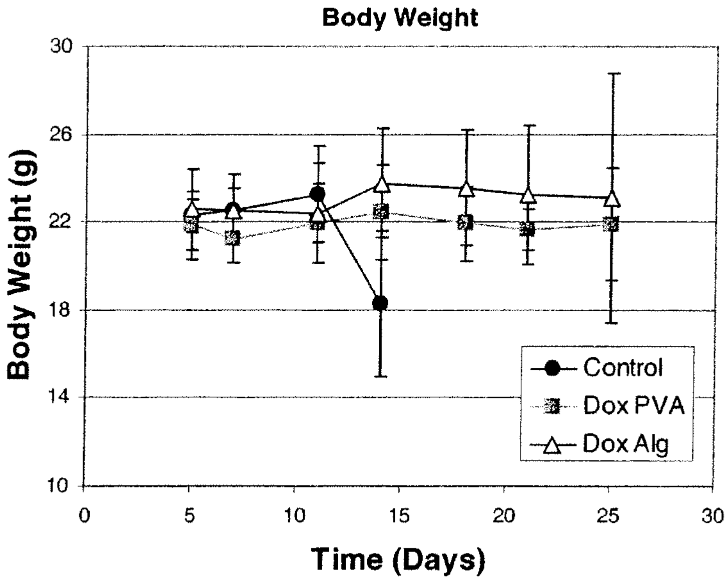


Figure 12

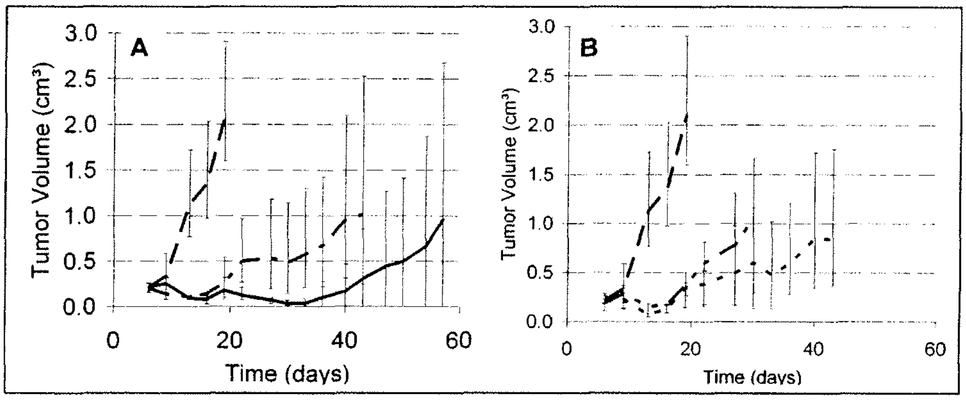


Figure 13

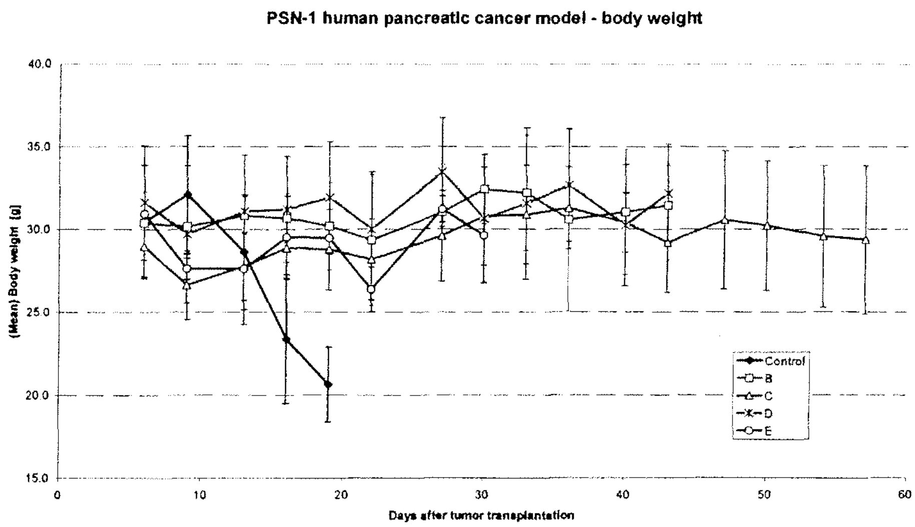


Figure 14

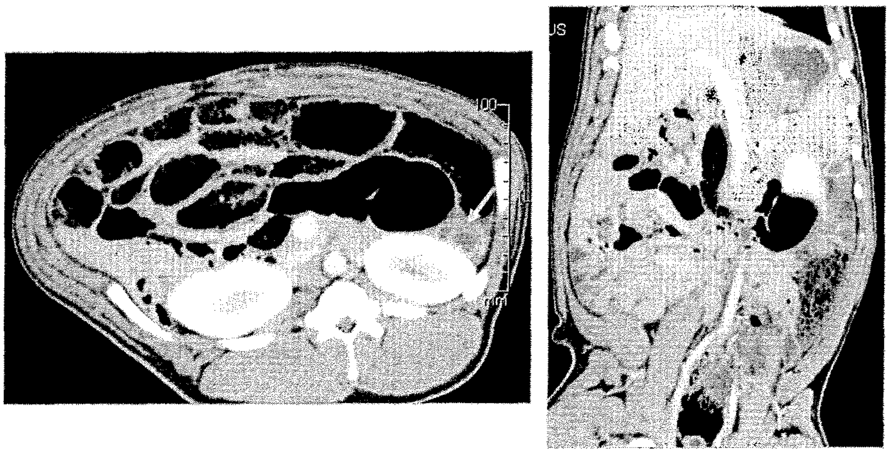

Figure 15

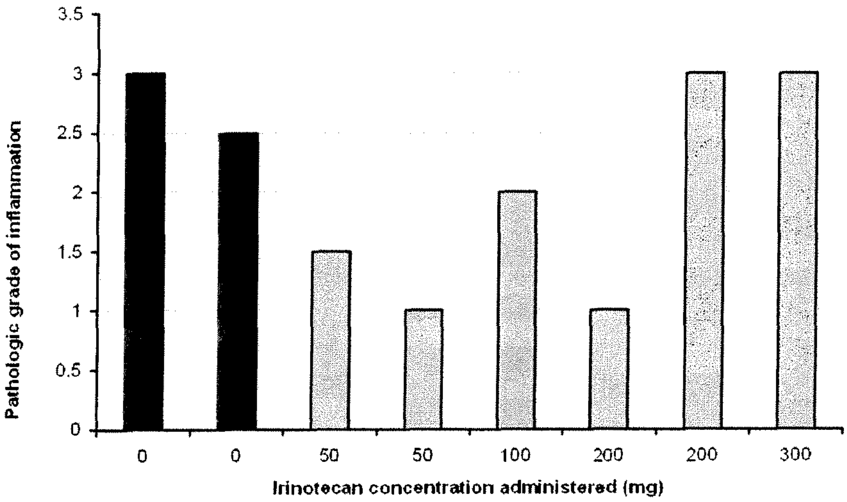


Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006027567 A
- WO 04071495 A
- WO 06027567 A
- WO 08128580 A
- US 4978713 A
- US 5508317 A
- US 5583163 A
- WO 0029481 A
- WO 9207885 A
- WO 9416748 A
- WO 9416749 A
- WO 9520407 A
- US 4224427 A
- WO 200709089 A
- GB 2008050722 W
- WO 2003022807 A
- WO 2004071495 A
- WO 200602756 A
- WO 2007090897 A


### Non-patent literature cited in the description


- **AHMEDIN JEMAL ; DVM ; PHD ; REBECCA SIEGE ; MPH ; ELIZABETH WARD ; PHD ; TAYLOR MURRAY ; JIAQUAN XU ; MICHAEL J. THUN.** *MD,MS. Cancer Statistics, 2007. CA Cancer J Clin,* 2007, vol. 57, 43-66
- **STEPHENS J ; KUHN J ; O'BRIEN J et al.** Surgical morbidity, mortality, and long-term survival in patients with peripancreatic cancer following pancreaticoduodenectomy. *Am J Surg.,* 1997, vol. 174, 600-604
- **ANDERSEN JR ; FRIIS-MÖLLER A ; HANCKE S ; RÖDER O ; STEEN J ; BADEN H.** A controlled trial of combination chemotherapy with 5-FU and BCNU in pancreatic cancer. *Scand J Gastroenterol,* 1981, vol. 16, 973-5
- **FREY C ; TWOMEY P ; KEEHN R ; ELLIOTT D ; HIGGINS G.** Randomized study of 5-FU and CCNU in pancreatic cancer: report of the Veterans Administration Surgical Adjuvant Cancer Chemotherapy Study Group. *Cancer,* 1981, vol. 47, 27-31
- **MALLINSON CN ; RAKE MO ; COCKING JB ; FOX CA ; CWYNARSKI MT ; DIFFEY BL et al.** Chemotherapy in pancreatic cancer: results of a controlled, prospective, randomised, multicentre trial. *BMJ,* 1980, vol. 281, 1589-91
- **MOERTEL CG ; FRYTAK S ; HAHN RG ; O'CONNELL MJ ; REITEMEIER RJ ; RUBIN J et al.** Therapy of locally unresectable pancreatic carcinoma: a randomized comparison of high dose (6000 rads) radiation alone, moderate dose radiation (4000 rads 1 5-fluorouracil), and high dose radiation 1 5-fluorouracil: the Gastrointestinal Tumour Study Group. *Cancer,* 1981, vol. 48, 1705-10
- Gastrointestinal Tumour Study Group. Radiation therapy combined with Adriamycin or 5-fluorouracil for the treatment of locally unresectable pancreatic carcinoma. *Cancer,* 1985, vol. 56, 2563-8
- **KLAASSEN DJ ; MACINTYRE JM ; CATTON GE ; ENGSTROM PF ; MOERTEL CG.** Treatment of locally unresectable cancer of the stomach and pancreas: a randomized comparison of 5-fluorouracil alone with radiation plus concurrent and maintenance 5-fluorouracil-an Eastern Cooperative Oncology Group study. *J Clin Oncol,* 1985, vol. 3, 373-8
- Gastrointestinal Tumour Study Group. Treatment of locally unresectable carcinoma of the pancreas: comparison of combined-modality therapy chemotherapy plus radiotherapy to chemotherapy alone. *J Natl Cancer Inst,* 1988, vol. 80, 751-5
- **BURRIS HA III ; MOORE MJ ; ANDERSEN J ; GREEN MR ; ROTHENBERG ML ; MODIANO MR et al.** Improvements in survival and clinical benefit with gemcitabine as first-line therapy for patients with advanced pancreas cancer: cancer: a randomized trial. *J Clin Oncol,* 1997, vol. 15, 2403-13
- **LORENZ M ; HEINRICH S ; STAIB-SEBLER E ; KOHNE CHO ; VLLILS J ; NORDLINGER B ; ENCKE A.** Regional chemotherapy in the treatment of advanced pancreatic cancer - is it relevant?. *Eur J Cancer.,* May 2000, vol. 36 (8), 957-65
- **NIRAG C. JHALA ; DARSHANA N. JHAL ; DAVID C. CHHIENG ; MOHAMAD A. ELOUBEIDI ; ISAM A. ELTOUM ; ISAM A. ELTOUM.** *Am J Clin Pathol,* 2003, vol. 120 (3), 351-367
- **ANTHONY J. DIMARINO, JR. ; ANTHONY J. DIMARINO ; STANLEY B. BENJAMIN.** *Gastrointestinal Disease: An Endoscopic Approach,* 2002, ISBN 1556425112, 1078

- **KENNETH J. ; CHANG, M.D. ; PHUONG T. ; NGUYEN, M.D. ; JAMES A. ; THOMPSON, M.D. ; THOMAS T. ; KUROSAKI, M.S. ; LINDA R. ; CASEY, M.D.** Phase I clinical trial of allogeneic mixed lymphocyte culture (cytoimplant) delivered by endoscopic ultrasound-guided fine-needle injection in patients with advanced pancreatic carcinoma. *Cancer,* vol. 88 (6), 1325-1335
- **OH HC ; SEO DW ; LEE TY ; KIM JY ; LEE SS ; LEE SK ; KIM MH.** New treatment for cystic tumours of the pancreas: EUS-guided ethanol lavage with paclitaxel injection. *Gastrointest Endosc,* April 2008, vol. 67 (4), 636-42
- **MATTSSON J ; NAREDI P ; HAFSTRÖM L ; PETERSON HI.** Intratumoural distribution of microspheres. *Anticancer Res,* July 1986, vol. 6 (4), 563-6
- **GUPTA PK ; HUNG, CT ; LAM FC.** Application of regression analysis in the evaluation of tumour response following the administration of adriamycin as a solution or via albumin microspheres to the rat. *J Pharm Sci.,* 1900, vol. 79 (7), 634-7
- **ALMOND BA ; HADBA AR ; FREEMAN ST ; CUEVAS BJ ; YORK AM ; DETRISAC CJ ; GOLDBERG EP.** Efficacy of mitoxantrone-loaded albumin microspheres for intratumoural chemotherapy of breast cancer. *J Control Release,* 28 August 2003, vol. 91 (1-2), 147-55
- **EMERICH DF ; SNODGRASS P ; LAFRENIERE D ; DEAN RL ; SALZBERG H ; MARSH J ; PERDOMO B ; ARASTU M ; WINN SR ; BARTUS RT.** Sustained release chemotherapeutic microspheres provide superior efficacy over systemic therapy and local bolus infusions. *Pharm Res.,* July 2002, vol. 19 (7), 1 052-60
- **LIU Z ; BALLINGER JR ; RAUTH AM ; BENDAYAN R ; WU XY.** Delivery of an anticancer drug and a chemosensitizer to murine breast sarcoma by intratumoural injection of sulfopropyl dextran microspheres. *J Pharm Pharmacol,* August 2003, vol. 55 (8), 1063-73
- **SUN S et al.** *EUS-guided interstitial chemotherapy in the pancreas Endoscopy,* 2007, vol. 39, 530-534